(19) Europäisches Patentamt
European Patent Office
Office européen des brevets

(11) Numéro de publication: **0 006 068**
**B1**

(12) **FASCICULE DE BREVET EUROPEEN**

(45) Date de publication du fascicule du brevet: **21.09.83**

(21) Numéro de dépôt: **79400357.4**

(22) Date de dépôt: **01.06.79**

(51) Int. Cl.³: **C 07 H 15/04,** C 07 H 15/12, C 07 H 15/20, A 61 K 31/70, G 01 N 33/50

(54) **Composés du type muramyl-peptide et médicaments les contenant.**

(30) Priorité: **05.06.78 FR 7816793**

(43) Date de publication de la demande:
**12.12.79 Bulletin 79/25**

(45) Mention de la délivrance du brevet:
**21.09.83 Bulletin 83/38**

(84) Etats contractants désignés:
**AT BE CH DE FR GB IT LU NL SE**

(56) Documents cités:
**FR - A - 2 292 486**
**FR - A - 2 355 505**
**FR - A - 2 358 159**
**FR - A - 2 368 282**
**FR - A - 2 369 292**

(73) Titulaire: **ANVAR Agence Nationale de Valorisation de la Recherche**
**13, rue Madeleine Michelis**
**F-92522 Neuilly-sur-Seine (FR)**

(72) Inventeur: **Lefrancier, Pierre**
**20, rue des Bleuets**
**F-91440 Bures sur Yvette (FR)**
Inventeur: **Parant, Monique**
**33, rue Croulebarbe**
**F-75013 Paris (FR)**
Inventeur: **Audibert, Françoise**
**44, rue Ybry**
**F-92200 Neuilly sur Seine (FR)**
Inventeur: **Chedid, Louis**
**16-18, rue Gaston de Caillavet**
**F-75015 Paris (FR)**
Inventeur: **Choay, Jean**
**130, Faubourg Saint-Honoré**
**F-75008 Paris (FR)**
Inventeur: **Lederer, Edgar**
**9, Boulevard Colbert**
**F-92330 Sceaux (FR)**

(74) Mandataire: **Gutmann, Ernest et al,**
**Cabinet Plasseraud 84, rue d'Amsterdam**
**F-75009 Paris (FR)**

**EP 0 006 068 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Composés du type muramyl-peptide et médicaments les contenant

L'invention est relative à de nouveaux composés du type 2-(2-acétamido-2-désoxy-3-O-D-glucopyranosyl)-alcanoyl-peptide, composés qui sont doués de propriétés biologiques et pharmacologiques de grande valeur. Plus particulièrement, l'invention concerne parmi ces composés ceux qui possèdent des propriétés immunorégulatrices, notamment d'adjuvants immunologiques non spécifiques, ces composés étant aptes, entre autres activités, à renforcer l'activité immunoprotectrice d'agents imunogènes de tous genres, naturels ou synthétiques.

L'invention concerne également les applications auxquelles les composés, objet de la présente demande, sont susceptibles de donner lieu, ainsi que les compositions particulières contenant de tels composés, plus particulièrement appropriées à la mise en oeuvre de ces applications.

Elle concerne aussi des réactifs biologiques, par exemple des adjuvants immunologiques standards, qui peuvent être constitués à l'aide des composés selon l'invention, notamment en vue d'étudier les éventuelles propriétés adjuvantes de substances à l'étude par comparaison à de tels adjuvants standards ou, au contraire, comme agent susceptible de s'opposer à certains effets liés à l'administration de substances immunosuppressives.

L'invention concerne plus particulièrement encore l'application des composés en question à l'amplification de l'effet immunogène de principes actifs de vaccins administrés à un hôte, animal ou humain. En conséquence, l'invention concerne également encore des compositions pharmaceutiques dont le principe actif est constitué par un au moins des composés définis ci-après, en association avec le véhicule pharmaceutique approprié au mode d'administration requis ou utilisable eu égard à la nature du principe vaccinant utilisé.

On sait que des efforts considérables sont depuis longtemps consacrés à la recherche d'agents doués de propriétés adjuvantes du type rappelé ci-dessus. Longtemps, ces efforts de recherche ont porté sur des extraits naturels, tels qu'ils peuvent être obtenus à partir de bactéries, notamment de mycobactéries. Bien que l'on ait ainsi pu obtenir des produits hautement actifs et ayant atteint un taux de purification élevé, un progrès décisif a été accompli par la mise au point de séries de molécules plus petites, désormais accessibles à la synthèse chimique. Un des composés les plus représentatifs de ces séries est constitué par la 2-(2-acétamido-2-désoxy-3-O-D-glucopyranosyl)-D-propionyl-L-alanyl-D-isoglutamine, encore dénommé plus simplement N-acétyl-muramyl-L-alanyl-D-isoglutamine, souvent dénommé MDP, de formule:

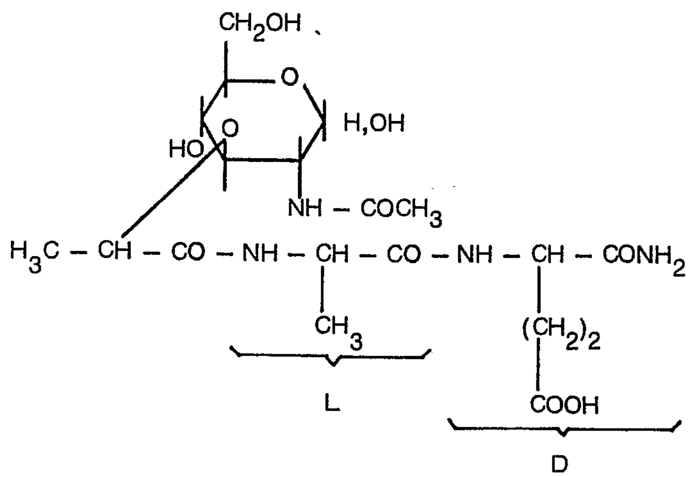

(Ce composé a fait l'objet d'un brevet français n° 74 22909/2.292.486).

Ainsi a-t-il été possible d'obtenir des séries de composés dépourvus des effets toxiques bien connus provoqués par les immunostimulants et dont l'activité d'adjuvant immunologique, très puissante pour certains d'entre eux, se manifeste lorsque ces composés sont administrés à un hôte, même en l'absence de tout support huileux, comme il était auparavant nécessaire pour permettre la manifestation des propriétés d'adjuvants immunologiques des extraits naturels, notamment obtenus à partir de mycobactéries.

Les études menées sur ces séries de composés ont permis de constater que la modification ou la substitution de certains groupements fonctionnels appartenant à diverses parties de la molécule, par exemple le remplacement du premier résidu d'aminoacide de la chaîne peptidique dans la N-acétyl-muramyl-L-alanyl-D-isoglutamine, pouvaint être effectuées sans que soit perdue l'activité adjuvante du composé initial.

A cet égard, on peut citer au titre de l'état de la technique également encore les brevets français n° 76 19236/2.355.505 et n° 76 31935/2.368.282, lesquels décrivent des composés de structures voisines de celles du MDP, mais qui ont en commun avec celui-ci un groupe isoglutamine.

Ainsi, l'activité adjuvante peut être préservée lors du remplacement du groupe proprionyle, substituant la position 2 du résidu glucopyranosyle par un autre groupe de type alcanoyle à l'endroit correspondant de la molécule. Enfin, le premier résidu aminoacyle, à savoir L-alanyle, peut être remplacé par d'autres résidus aminoacyles, tels que glycyle ou de préférence L-séryle. On peut également le remplacer par un autre résidu aminoacyle tel que par exemple L-prolyle, L-thréonyle ou L-valyle.

Par contre, d'autres parties de la molécule ne peuvent être modifiées de façon profonde sans que soit alors perdue, au moins dans une grande mesure, l'activité adjuvante de l'ensemble de la molécule. A cet égard, la présence dans la chaîne peptidique d'un groupe dérivé de l'acide glutamique, au titre du second résidu aminoacyle de la chaîne, est à ce jour critique pour le maintien de l'activité adjuvante. En outre, il est admis que les composés très actifs en tant qu'adjuvants immunologiqes sont ceux pour lesquels le groupe $\alpha$-carboxylique du résidu glutamique a été transformé en groupe amide lorsqu'ils sont utilisés sous forme d'émulsion eau-dans-l'huile. L'importance de la configuration carboxamide du groupe $\alpha$-carboxylique du résidu glutamique a été soulignée par divers auteurs. A cet égard, on peut rappeler par exemple les observations d'Arlette ADAM et coll. (Biochemical & Biophysical Research Communications, vol. 72, No. 1, 1976) qui ont par exemple mis en évidence l'activité adjuvante dans l'huile moins forte de l'ester diméthylique de l'acide N-acétyl-muramyl-L-alanyl-D-glutamique (lequel présente par ailleurs d'autres propriétés biologiques du plus grand intérêt) que celle de l'ester $\gamma$-méthylique de la N-acétyl-muramyl-l-alanyl-D-isoglutamine. Ces derniers composés sont également l'objet du brevet français n° 77 02646/2.369.292.

Des observations semblables ont été faites par une équipe de chercheurs japonais, comme il est encore remarqué par exemple dans la publication de Shozo KOTANI et coll. (Biken Journal, vol. 19, 9—13, 1976).

Les composés déjà décrits dans l'état de la technique, parmi lesquels ceux dont le résidu glutamique est $\alpha$-carboxamidé, sont cependant susceptibles de présenter *in vivo* un certain effet pyrogène, notamment lorsqu'ils sont administrés à des doses élevées. Des études effectuées dans ce domaine par différentes équipes on fait apparaître qu'il existait une corrélation apparente entre l'effet adjuvant des substances les plus actives et leur pyrogénicité possible dans certaines conditions expérimentales. Shozo KOTANI et coll. ont émis l'hypothèse que cette pyrogénicité pouvait éventuellement être attribuée à la parenté entre les composés étudiés et les fragments de peptido-glycane qui peuvent être obtenus à partir de bactéries gram-positif (dans la publication déjà susmentionée).

Ces auteurs formulent l'hypothèse qu'il pourrait exister une éventuelle relation centre les mécamismes intervenant au niveau des réponses immunologiques des mammifères, sous l'effet d'une stimulation antigénique, et ceux intervenant au niveau de la régulation de la température du corps ou de la réponse fébrile. Ils émettent aussi l'idée que certaines cibles des N-acétyl-muramyl-peptides sont impliquées dans les deux mécanismes. En effet, ces auteurs ont constaté que, parmi les composés qu'ils ont testés, les plus adjuvants étaient aussi les plus pyrogènes, et qu'à l'inverse les moins adjuvants étaient aussi les moins pyrogènes.

Cette règle n'a effectivement pas été mise réellement en défaut jusqu'à ce jour, même si certains produits déjà décrits présentent un très faible niveau de pyrogénicité aux doses et dans les conditions de l'expérimentation. Il en est ainsi, par exemple, du diamide de l'acide N-acétyl-muramyl-L-alanyl-D-glutamique (décrit dans le brevet français n° 2.358.159). Cependent, ce produit, s'il possède un index thérapeutique particulièrement favorable, est toutefois moins actif que la N-acétyl-muramyl-L-alanyl-D-isoglutamine.

Le développement des études dans ce domaine particulier de la technique, qui a conduit aux composés nouveaux qui sont l'objet de la présente demande, a permis d'aboutir à un certain nombre de conclusions surprenantes. En effet, il est possible d'obtenir des composés adjuvants très puissants, comportant une seule fonction amide substituant le groupe $\gamma$-carboxylique du résidu glutamique des dérivés du genre en question, à la condition cependant que le groupe $\alpha$-carboxylique du résidu glutamique soit également substitué de façon déterminée, cette activité adjuvante étant susceptible de se manifester aussi bien en solution aqueuse qu'en émulsion eau dans l'huile. Ce résultat est d'autant plus inattendu qu'il est bien connu que la N-acétyl-muramyl-L-alanyl-D-glutamine (comportant donc une fonction amide sur le groupe carboxylique en $\gamma$ du groupe glutamyle) n'est que très faiblement adjuvante *in vivo*, lorsqu'elle est administrée sous la forme d'une émulsion eau dans l'huile.

De même, l'apparente corrélation qui a été remarquée entre les actions adjuvantes et pyrogènes des composés connus paraît désormais sérieusement mise en défaut par les résultats expérimentaux obtenus dans le cadre de la présente invention. Il en est tout particulièrement ainsi des composés selon l'invention dont certains sont caractérisés par un degré d'apyrogénicité jusqu'à présent jamais atteint.

Ces constatations s'appliquent aux produits selon l'invention qui, comme ceux de l'état de la technique, sont constitués d'un composé du type 2-(2-amino ou acyl-amido-2-désoxy-3-O-D-glucopyranosyl)-alcanoyl-peptide présentant, le cas échéant, les substitutions et remplacements qui ont été évoqués plus haut, ces produits étant néanmoins caractérisés par les présences simultanées d'une fonction amide (—$CONH_2$) sur le groupe $\gamma$-carboxylique et d'une fonction ester sur le groupe $\alpha$-carboxylique du résidu glutamique.

L'invention concerne notamment les nouveaux composés répondant à la formule générale

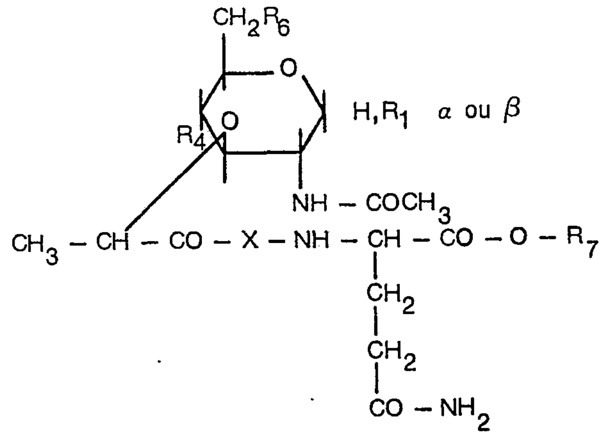

dans laquelle les substituants $R_1$, $R_4$, $R_6$, X et $R_7$ ont respectivement les significations suivantes:

$R_1$ est —$NH_2$, —OH, —NY ou —OY, Y représentant un groupe substitué ou non par un groupe amino, ce groupe étant choisi parmi alcoyle, aryle ou alcoyl-aryle portant un maximum de 10 atomes de carbone;

$R_4$ est hydroxyle, acyloxy ou alcoxy ayant au plus 4 atomes de carbone, ou monosuccinyle;

$R_6$ est —$NH_2$, —OH, —NHZ ou —OZ, Z étant un radical linéaire ou ramifié formé par acyle ou alkyle contenant de 1 à 90 atomes de carbone et portant, le cas échéant, des substituants hydroxyle, carboxyle, carbonyle, amino, cyclopropyle ou méthoxyle;

X est un résidu aminoacyle du groupe comprenant: L-alanyle, L-arginyle, L-asparagyle, L-aspartyle, L-cystéinyle, L-glutaminyle, L-glutamyle, glycyle, L-histidyle, L-hydroxy-propyle, L-isoleucyle, L-leucyle, L-lysyle, L-méthionyle, L-phénylalanyle, L-propyle, L-séryle, L-thréonyle, L-tryptophanyle, L-tyrosyle et L-valyle,

$R_7$ est un groupe alcoyle linéaire ou ramifié, aryle ou alcoyl-aryle comprenant au plus 10 atomes de carbone.

Parmi les composés de formule générale I, il en est de particulièrement avantageux. Ci-dessous sont indiquées les différentes significations des éléments variables de la formule I correspondant à des structures préférées.

Dans cette formule, le deuxième groupe aminoacyle de la chaîne peptidique liée au résidu de type muramyle est le résidu D-glutamyle. Le premier groupe aminoacyle (désigné par X) peut, par contre, être choisi parmi les différents résidus aminoacyles mentionnés ci-dessus. Parmi les composés de formule I, on préfère ceux dans lesquels le premier résidu aminoacyle est L-alanyle. Un deuxième type de composés préférés est celui dans lequel cet aminoacyle est L-séryle. Un autre type de composés préférés est celui dans lequel cet aminoacyle est glycyle.

Sont également avantageux les composés dans lesquels le premier résidu aminoacyle est L-prolyle, L-thréonyle ou L-valyle.

Le groupe en $\alpha$ du résidu D-glutamyle est de préférence un ester à chaîne carbonée comprenant de 1 à 4 atomes de carbone.

Une forme préférée est constituée par le cas où $R_7$ est soit —$CH_3$, soit —$C_2H_5$ ou $C_3H_7$.

Une autre forme préférée est constituée par les composés dans lesquels le radical $R_7$ comprend 4 carbones.

La liaison glycosidique de la partie saccharidique dans les produits selon l'invention peut se présenter sous les formes anomères $\alpha$ ou $\beta$. Le résidu osidique peut recevoir également différents substituants dont la littérature antérieure, relative aux agents adjuvants du type muramyl-peptide, a donné un certain nombre d'exemples sur ses fonctions hydroxyle qui peuvent être estérifiées ou éthérifiées.

Dans la formule générale des produits selon l'invention, les substituants du cyle glucopyranoside ont été désignés par $R_1$, $R_2$, $R_4$ et $R_6$. Les différentes positions ne présentent pas les mêmes possibilités de substitution, la position 6 étant celle pour laquelle la plus grande latitude est offerte.

Des composés préférés sont ceux pour lesquels un ou plusieurs des substituants $R_1$, $R_4$ et $R_6$, indépendamment les uns des autres ou simultanément, sont un hydroxyle.

Des composés avantageux sont également ceux pour lesquels $R_4$ correspond aux esters monosuccinique ou acétique.

Des composés préférés sont ceux pour lesquels $R_6$ est une fonction amine, ou encore un ester, dont le résidu acyle contient de 1 à 6 atomes de carbone, en particulier les esters acétique ou monosuccinique. $R_6$ est aussi avantageusement l'ester correspondant aux acides mycolique (environ $C_{80}$ à $C_{90}$) ou corynomycolique ($C_{32}$).

Des composés préférés selon l'invention sont les esters méthylique, éthylique, propylique, hexylique ou décylique de la N-acétyl-muramyl-L-alanyl-D-glutamine. Un composé particulièrement préféré est l'ester butylique de la N-acétyl-muramyl-L-alanyl-D-glutamine.

D'autres composés préférés sont ceux de formule I dans laquelle les divers substituants correspondant à ceux donnés dans le tableau suivant.

| $R_1$ | $R_4$ | $R_6$ | X | $R_7$ |
|---|---|---|---|---|
| OH | OH | OH | L-Ala | $C_2H_5$ |
| d° | d° | d° | Gly | $CH_3$ |
| d° | d° | d° | d° | $C_4H_9$ |
| d° | d° | d° | d° | $C_6H_{13}$ |
| d° | d° | d° | d° | $C_{10}H_{21}$ |
| d° | d° | d° | L-Ser | $CH_3$ |
| d° | d° | d° | d° | $C_4H_9$ |
| d° | d° | d° | d° | $C_6H_{13}$ |
| d° | d° | d° | d° | $C_{10}H_{21}$ |
| d° | d° | d° | L-Val | $CH_3$ |
| d° | d° | d° | d° | $C_4H_9$ |
| d° | d° | d° | d° | $C_6H_{13}$ |
| d° | d° | d° | d° | $C_{10}H_{21}$ |
| d° | d° | Corynomy-coloyl | d° | $CH_3$ |
| d° | d° | d° | d° | $C_4H_9$ |
| d° | d° | d° | d° | $C_{10}H_{21}$ |
| d° | d° | d° | Ser | $CH_3$ |
| d° | d° | d° | d° | $C_4H_9$ |
| d° | d° | d° | d° | $C_{10}H_{21}$ |
| d° | d° | Mycoloyl | L-Ala | $CH_3$ |
| d° | d° | d° | d° | $C_4H_9$ |
| d° | d° | d° | d° | $C_{10}H_{21}$ |
| d° | d° | d° | Ser | $CH_3$ |
| d° | d° | d° | d° | $C_4H_9$ |
| d° | d° | d° | d° | $C_{10}H_{21}$ |
| $C_6H_4—NH_2$ | d° | OH | L-Ala | $CH_3$ |

Table cont.

| $R_1$ | $R_4$ | $R_6$ | X | $R_7$ |
|---|---|---|---|---|
| $C_6H_4—NH_2$ | OH | OH | L-Ala | $C_4H_9$ |
| d° | d° | d° | d° | $C_{10}H_{21}$ |
| OH | $OCH_3$ | OH | L-Ala | $CH_3$ |
| d° | d° | d° | d° | $C_4H_9$ |
| OH | $OCH_3$ | OH | L-Ala | $C_{10}H_{21}$ |
| d° | d° | d° | L-Ser | $CH_3$ |
| d° | d° | d° | d° | $C_4H_9$ |
| d° | d° | d° | d° | $C_{10}H_{21}$ |
| d° | OH | $OCH_3$ | L-Ala | $CH_3$ |
| d° | d° | d° | d° | $C_4H_9$ |
| d° | d° | d° | d° | $C_{10}H_{21}$ |
| d° | d° | d° | L-Ser | $CH_3$ |
| d° | d° | d° | d° | $C_4H_9$ |
| d° | d° | d° | d° | $C_{10}H_{21}$ |
| d° | $OCH_3$ | $OCH_3$ | L-Ala | $CH_3$ |
| d° | d° | d° | d° | $C_4H_9$ |
| d° | d° | d° | d° | $C_{10}H_{21}$ |
| d° | d° | d° | L-Ser | $CH_3$ |
| d° | d° | d° | d° | $C_4H_9$ |
| d° | d° | d° | d° | $C_{10}H_{21}$ |
| d° | OH | $OCH_3$ | L-Ala | $CH_3$ |
| d° | d° | d° | d° | $C_4H_9$ |
| d° | d° | d° | d° | $C_{10}H_{21}$ |
| d° | d° | $OC_{10}H_{21}$ | d° | $CH_3$ |
| d° | d° | d° | d° | $C_4H_9$ |
| d° | d° | d° | d° | $C_{10}H_{21}$ |
| d° | d° | d° | L-Ser | $CH_3$ |

Table cont.

| $R_1$ | $R_4$ | $R_6$ | X | $R_7$ |
|---|---|---|---|---|
| OH | OH | $OC_{10}H_{21}$ | L-Ser | $C_4H_9$ |
| d° | d° | d° | d° | $C_{10}H_{21}$ |
| d° | d° | $C_4H_5O_4$ | L-Ala | $CH_3$ |
| d° | d° | d° | d° | $C_4H_9$ |
| d° | d° | d° | d° | $C_{10}H_{21}$ |
| d° | d° | d° | L-Ser | $CH_3$ |
| d° | d° | d° | d° | $C_4H_9$ |
| d° | d° | d° | d° | $C_{10}H_{21}$ |

Les produits selon l'invention sont préparés par synthèse, certains des composés utilisés dans ces synthèses pouvant provenir de produits naturels.

La synthèse de ces molécules de nature glycopeptidique, dont la séquence comprend un reste dipeptidique fixé à un résidu d'acide N-acétyl-muramique, ou d'un analogue ou dérivé de celui-ci tel qu'indiqué ci-dessus, est réalisée selon des méthodes utilisés classiquement en synthèse peptidique ou saccharidique. De telles méthodes ont été amplement décrites dans la littéature antérieure, ainsi que dans des demandes de brevets français, notamment n° 76 06820, 76 06821, 76 21889 et 77 02646. 02646.

Ces synthèses peuvent être faites par le couplage d'un dérivé de l'acide muramique, ou d'un analogue de celui-ci, soit successivement avec un dérivé du premier acide aminé, puis du second acide aminé, soit avec un dérivé dipeptidique, les groupements de protection temporaire étant finalement éliminés. Dans une variante, ces synthèses conduisent à l'obtention de dérivés glycopeptidiques dont la fonction carboxylique est libre et peut être alors substituée par un groupement ester ($R_7$). Le choix de la séquence réactionnelle est guidé principalement par des raisons de commodité, de rendement et d'obtention de produits homogènes, notamment stéréochimiquement.

On a donné ci-après, de façon succincte, les principales indications relatives à différentes opérations qui peuvent être mises en oeuvre pour synthétiser les produits répondant à la formule I, d'abord en envisageant séparément chaque étape, puis en indiquant quelques séquences réactionnelles types préférées.

1°) Préparation de l'acide muramique, de ses analogues ou de leurs dérivés.

La préparation de tels produits peut se faire à partir de composés décrits dans des publications antérieures. Eventuellement, pour ceux dont la préparation n'apparaîtrait pas expressément dans la littérature, ils peuvent être obtenus selon les modes d'obtention des dérivés correspondants, utilisés classiquement en chimie des oligosaccharides.

a) Préparation de l'acide muramique ou de ses analogues.

Pour obtenir les analogues de l'acide N-acétyl-muramique de formule

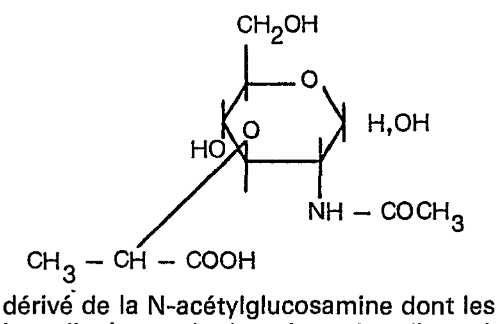

il est possible de partir d'un dérivé de la N-acétylglucosamine dont les hydroxyles en position 1, 4 et 6 sont bloqués de façon traditionnelle. Le mode de préparation d'un tel dérivé, le benzyle-2-acétamido-4,6-O-benzylidène-2-déoxy-D-glucopyranoside, est décrit notamment par P. H. GROSS et R. W. JEANLOZ (J. Org. Chem. 1967, *32,* 2761).

La formation de l'acide N-acétyl-muramique ou d'un de ses analogues peut être réalisée de la manière décrite dans la demande de brevet français n° 74 22909 reprenant la méthode décrite par OZAWA et JEANLOZ (J. Org. Chem. 1965, *30*, 448).

Cette formation comprend par exemple la préparation d'un sel de sodium de l'hydroxyle en position 3 et la condensation ultérieure du dérivé sodé avec le sel ou l'ester de l'acide $\alpha$ halogéné tel que l'acide chloro-2-propionique pour reprendre le cas de la demande de brevet indiquée précédemment. Le composé halogéné utilisé de forme L peut être préparé suivant la méthode décrite par SINAY et al (J. Biol. Chem., 1972, *247*, 391).

Lorsqu'on utilise un ester d'acide halogéné, afin de pouvoir procéder à la condensation peptidique ultérieure, la fonction carboxylique peut être libérée par une hydrolyse appropriée.

b) Substitution sur le résidu saccharidique.

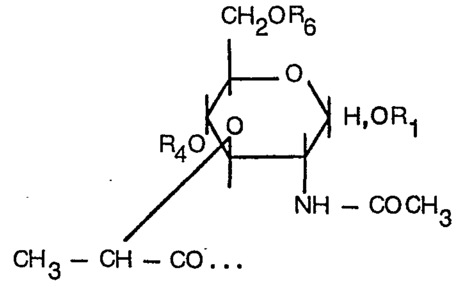

Les substitutions en position 1, 4 et 6 peuvent être réalisées par des méthodes qui ont été décrites antérieurement et qui sont traitionnelles dans la chimie des sucres. Lorsque les substituants envisagés sont différents les uns des autres, on procédera à autant de réactions de substitution successives qu'il y a de substituants distincts. Au cours de ces réactions, les positions ne devant pas être substituées ou celles qui doivent faire ultérieurement l'objet d'une autre substitution sont protégées temporairement par des groupes de blocage selon les modes habituels.

Les groupes de blocage initialement présents, dans le cas où l'on part, comme indiqué précédemment, de benzyl-2-acétamido-4,6,-O-benzylidène-2-déoxy-D-glucopyranoside, sont éliminés par exemple par action de l'acide acétique (à 60% 1 heure à reflux) et hydrogénation catalytique, comme décrit par exemple par MERSER et al. (Biochem. Biophys. Res. Commun., 1974, *466*, 1316), ou par hydrogénation catalytique comme décrit par exemple par LEFRANCIER et al. (Int. J. Peptide Protein Res., 1977, *9*, 249).

Les modes de substitution sont ceux traditionnellement utilisés. Pour obtenir les dérivés acylés, on opère à l'aide d'un agent acylant correspondant au substituant que l'on souhaite introduire (anhydride, chlorure d'acyle, etc.).

Les positions 1, 4, 6 ne sont pas équivalentes pour ce qui concerne leur réactivité. La position en $C^6$ est la plus facile à substituer, aussi, lorsque seule cette position doit être substituée, il est possible d'opérer sans bloquer les autres positions, avec une quantité d'agent de substitution équivalente à celle nécessaire pour la substitution d'une seule position.

Un exemple particulier de mode de préparation des dérivés substitués en position 6 est donné dans l'article de KUSUMOTO et al. (Tetrahedron Letters, 1976, *47*, 4237).

Les substitutions sur le résidu osidique peuvent être effectuées avant ou après la fixation de la chaîne peptidique ou des fragments de celle-ci.

2°) Préparation de la chaîne peptidique

La synthèse du reste dipeptidique est réalisée suivant les méthodes classiques de la synthèse peptidique. A titre d'exemple, on peut utiliser les méthodes d'activation des carboxyles, comme la méthode dite des anhydrides mixtes. De façon avantageuse, la synthèse peptidique est réalisée à l'aide d'un composé du type carbodiimide tel que le N,N'-dicyclo-hexylcarbodiimide ou des carbodiimides équivalents. On trouvera une revue des modes de synthèse peptidique traditionnels dans J.H. JONES, Chemistry and Industry, 723 (1974). On peut aussi se reporter aux demandes de brevets français déjà citées, ou encore aux demandes suivantes: 75 29624, 76 06819, 76 06820, 76 06821, 76 21889, 77 02646, et à l'article de LEFRANCIER et al. (Int. J. Peptide Protein Res., 1977, *9* 249).

Les substitutions de la fonction carboxyle du résidu D-glutaminyle par un groupement ester ($R_7$) sont avantageusement réalisées sur un dérivé de la D-glutamine, avant son couplage avec un dérivé de la L-alanine. Toutefois, il peut être également avantageux d'opérer cette substitution sur un dérivé dipeptidique. Dans les deux cas, la réaction se fait, par exemple, selon la technique décrite par WANG et al. (J. Org. Chem., *42* (1977), 1286).

Séquences de synthèse de glycopeptides de formule I

Le produit de départ est un dérivé (1), $R_1$ représentant un radical benzyle glycoside, préparé comme décrit par GROSS et JEANLOZ (J. Org. Chem., 1967, *32*, 2759). Pour obtenir le composé

semblable dans lequel $R_1$ est un groupement autre que benzyle, on peut utiliser la méthode de préparation des $\alpha$ ou $\beta$-glucosides décrite dans ce même article, ou toute méthode connue pour de telles préparations en chimie des oligosaccharides. Les dérivés de formule (2) peuvent être obtenus à partir des précédents suivant la méthode décrite par OZAWA et JEANLOZ (J. Org. Chem., 1965, *30,* 448), au moyen d'un acide L-$\alpha$-chloropropionique.

Les dérivés de formule (2) peuvent être couplés avec un dérivé dipeptidique de formule générale $H—X—D—Glu(NH_2)—O—R_7$ chlorhydrate.

Ces divers dérivés peptidiques sont préparés suivant des méthodes décrites par LEFRANCIER et al. (Int. J. Peptide Protein Res., 1977, *9,* 249; 1978, *11*, 289, et 1979 soumis à publication). Les méthodes de couplage utilisées pour obtenir les dérivés glycopeptidiques de formule (3) sont également décrites dans les articles précédemment cités. Toutefois, aussi bien dans la synthèse des dérivés dipeptidiques que dans celle des dérivés de formule (3), toute méthode de couplage peut être utilisée.

L'hydrogénation catalytique des composés de formule (3) est effectuée classiquement (LEFRANCIER et al., 1977, référence citée) pour aboutir aux composés de formule (4).

Dans une variante, les dérivés de formule (3) subissent une débenzylidénation sélective telle que décrite par MERSER et al. (Biochem. Biophys. Res. Commun., 1975, *66,* 1316) pour donner les dérivés de formule (5). L'acylation sélective de l'hydroxyle primaire en position 6 du résidu saccharidique peut alors se faire directement, notamment par action d'un faible excès de l'anhydride d'un acide carboxylique. On obtient des dérivés de formule (6).

Les dérivés de la formule (6) peuvent être synthétisés suivant une séquence totalement différente (schéma II, semblable à celle mise au point par KUSUMOTO et al. (Tetrahedron Letters, 1976, *47,* 4237), à partir de la tosylation spécifique de l'alcool primaire du résidu saccharidique.

Dans une autre variante, les dérivés de formule (5) sont diacylés sur les deux hydroxyles en position 4 et 6 du résidu saccharidique, notamment par action d'un excès de l'anhydride d'un acide carboxylique pour donner des composés de formule (7).

Dans une variante pour laquelle la fonction carboxyle du résidu D-glutamine est libre ($R_7 = OH$) dans les dérivés de formule (3), (5), un groupement ester ($R_7$) est introduit a posteriori selon la technique de WANG et al. (J. Org. Chem., *42,* 1977, 1286), pour obtenir les dérivés de formules (4), (6), (7) (Schéma I). La même séquence réactionnelle est applicable à l'obtention des dérivés de formule (4) (Schéma II).

## SCHEMA (I)

SEQUENCES DE SYNTHESE DE COMPOSES GLYCOPEPTIDIQUES

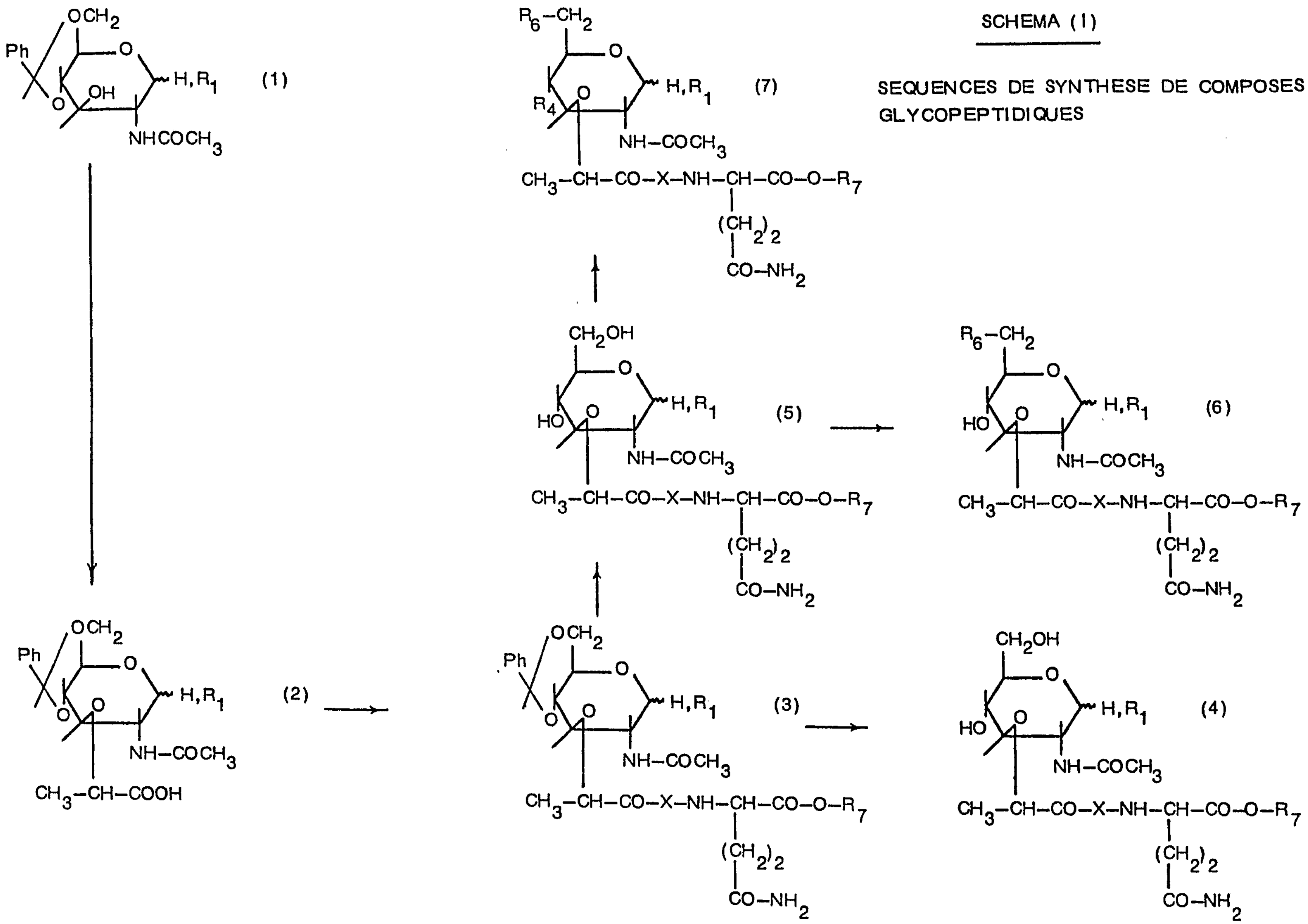

(1) (2) (3) (4) (5) (6) (7)

## SCHEMA (II)

SEQUENCE DE SYNTHESE DE DERIVES GLYCOPEPTIDIQUES CORRESPONDANT A LA FORMULE GENERALE ET DONT L'HYDROXYLE EN C6 DE RESIDU SACCHARIDIQUE EST ACYLE PAR UN ACID GRAS A LONGUE CHAINE

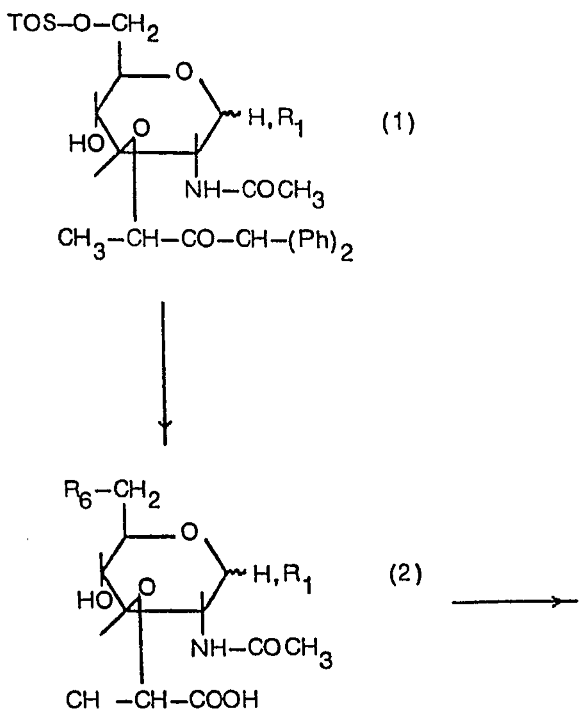

(1)

(2)

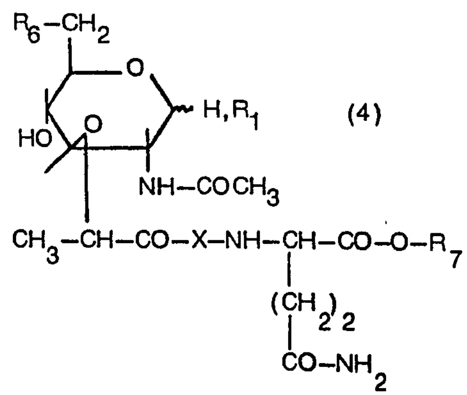

(4)

L'invention est aussi relative à des modes d'utilisation des composés répondant aux définitions précédentes, notamment en tant que réactif ou en tant que substance active dans des compositions pharmaceutiques.

L'invention concerne des réactifs biologiques, par exemple des adjuvants immunologiques standards, qui peuvent être constitués à l'aide des composés selon l'invention, notamment en vue d'étudier les éventuelles propriétés adjuvantes de substances à l'étude, par comparaison à de tels adjuvants standards ou, au contraire, comme agent susceptible de s'opposer à certains effets liés à l'administration de substances immunosuppressives.

Plus particulièrement, l'invention est relative à des médicaments renfermant à titre de principe actif au moins l'un des composés selon l'invention, ce médicament étant applicable en tant que régulateur de la réponse immunitaire du sujet auquel il est administré.

Ces médicaments sont notamment applicables lorsque l'on recherche un renforcement de la réponse immunitaire à tout agent immunogène. De tels agents immunogènes peuvent être naturels ou synthétiques, et nécessitent l'utilisation d'un agent stimulant le système immunitaire, soit que l'agent immunogène soit faible par nature, soit qu'il soit fort et puisse être utilisé à très faible dose, ou encore que le caractère immunogène ait été amoindri, par exemple au cours de modifications ou purifications antérieures. De façon générale, l'utilisation des composés immunorégulateurs selon l'invention est utile chaque fois que l'agent immunogène ne permet pas d'induire une réponse suffisante.

L'invention concerne plus particulièrement encore l'application des composés en question à l'amplification de l'effet immunogène de principes actifs de vaccins administrés à un hôte, animal ou humain, notamment dans le cas où ces principes vaccinants appartiennent aux catégories d'agents immunogènes rappelées ci-dessus. En conséquence, l'invention concerne également encore des compositions pharmaceutiques dont le principe actif est constitué par un au moins des composés selon l'invention, en association avec le véhicule pharmaceutique approprié au mode d'administration requis ou utilisable eu égard à la nature du principe vaccinant utilisé.

L'invention s'applique en particulier à ceux des agents vaccinants dont le caractère immunogène est fort mais qui sont d'une utilisation difficile en temps normal en raison d'une trop grande toxicité ou d'effets secondaires indésirables. Il a été confirmé que les agents adjuvants selon l'invention étaient capables de compenser efficacement la perte de l'effet immunogène qui résulterait normalement d'une dilution ou d'une diminution des doses utilisées, notamment dans le but de réduire la toxicité ou les effets secondaires susdits dans une proportion correspondante, et ce sans influencer défavorablement ces derniers phénomènes.

On constate les mêmes effets dans le cas d'agents vaccinants forts dont on a réduit le caractère immunogénique, notamment par des purifications poussées, dans la mesure où cela s'avère nécessaire à la diminution correspondante de leurs effets toxiques ou secondaires néfastes. Tel est en particulier le cas des principes vaccinants constitués par des anatoxines bactériennes ou virales ou, d'une façon générale, des principes vaccinants constitués par une partie seulement des constituants initialement contenus dans les bactéries ou virus contre lesquels une protection est recherchée.

D'une façon générale, l'invention s'applique à tout antigène ayant subi des transformations chimiques ou physiques visant à éliminer ou modifier les parties de l'antigène qui sont responsables de ses effets secondaires nuisables tout en conservant les parties qui sont à l'origine de ses propriétés immunogènes. C'est à de type d'immunogènes faibles que se rattachent par exemple les principes constitués par les "sous-unités" dérivées de virus de la grippe, et qui ne retiennent de ceux-ci que les hémagglutinines et les neuraminidases, à l'exclusion des nucléoprotéines et autres constituants nucléotidiques des virus dont elles sont dérivées. Ceci s'applique également à certaines anatoxines, telles que celles par exemple de la diphtérie ou du tétanos, lesquelles, on le sait, peuvent être constituées par des substances solubles, telles qu'obtenues par l'action simultanée du formol et de la chaleur sur des toxines bactériennes dérivées des bactéries correspondantes.

L'invention concerne encore l'application des composés selon l'invention, et notamment de ceux pour lesquels $R_7$ contient 4 carbones ou plus, pour le traitement des maladies infectieuses. Dans cette application, il faut remarquer que les produits selon l'invention se distinguent clairement des antibiotiques habituellement utilisés. Les produits selon l'invention, contrairement aux antibiotiques, n'ont pas d'effet bactéricide ou bactériostatique *in vitro*. Par contre, ils peuvent activer les macrophages isolés *in vitro* et leur action *in vivo* est manifeste comme on le verra dans les exemples d'essais pharmacologiques. Contrairement aux antibiotiques encore, l'action n'est pas limitée à certaines variétés de micro-organismes. Ceci s'explique par le fait que leur activité n'est pas directe mais se développe par l'intermédiaire des mécanismes de défense immunitaire non spécifique de l'hôte, mécanismes que leur administration stimule et amplifie. Cette différence d'action par rapport aux antibiotiques rend ces produits d'autant plus intéressants qu'ils peuvent être utilisés contre des germes pathogènes devenus résistants aux antibiotiques.

Le mode d'action des produits selon l'invention les rapproche des composés anti-infectieux connus tels que le BCG ou les lipopolysaccharides et comme tels peuvent être employés avec succès pour le traitement des infections sans présenter les inconvénients, notamment de toxicité, qui limitent ou interdisent l'utilisation des LPS ou du BCG.

Les produits selon l'invention peuvent en particulier être utilisés pour combattre de façon non

spécifique des maladies causées par des micro-organismes comme les *Klebsiella,* les *Pseudomonas,* les staphylocoques, etc.

Les applications indiquées précédemment à titre d'exemples ne sont pas exclusives des autres applications mettant en jeu les propriétés immunorégulatrices des composés selon l'invention. On peut encore citer à titre d'exemple leur action de renforcement au niveau de l'immunisation spécifique de l'hôte à l'égard d'antigènes parasitaires, la restauration de l'immunocompétence de l'hôte, lorsque celle-ci a un niveau inférieur à la normale, notamment lorsque celle-ci a été endommagée par les antigènes ou parasites eux-mêmes, ou sous l'effet d'une chimiothérapie, d'une radiothérapie, ou de tout autre traitement ayant une action immunosuppressive.

Les compositions pharmaceutiques selon l'invention, de façon générale, sont utiles pour le traitement ou la prévention de maladies infectieuses d'origine bactérienne ou parasitaire, ou pour l'inhibition d'affections tumorales.

Les adjuvants selon l'invention peuvent être administrés à l'hôte — animal ou être humain — de toute façon appropriée à l'obtention de l'effet désiré. Les administrations du principe immunorégulateur, notamment adjuvant, et de l'agent immunogène, notamment de l'antigène vaccinant, peuvent être envisagées simultanément ou séparément, dans ce dernier cas éventuellement décalées dans le temps, éventuellement encore par des voies d'administration semblables ou différentes (par exemple voies parentérale et orale respectivement ou vice versa).

L'invention concerne naturellement aussi les diverses compositions pharmaceutiques auxquelles les composés selon l'invention peuvent être incorporés, le cas échéant en association avec d'autres substances actives. En particulier, les composés I sont avantageusement associés à des agents immunogènes, qu'il s'agisse par exemple d'agents immunogènes utilisés à de très faibles doses, ou d'agents immunogènes faibles.

Des compositions pharmaceutiques avantageuses sont constituées par des solutions, suspensions ou forme liposome injectables, contenant une dose efficace d'au moins un produit selon l'invention. De préférence, ces solutions, suspensions ou forme liposome sont réalisées dans une phase aqueuse stérilisée isotonique, de préférence saline ou glucosée.

L'invention concerne plus particulièrement de telles suspensions, solutions ou forme liposome qui sont aptes à être administrées par injections intradermiques, intramusculaires ou sous-cutanées, ou encore par scarifications.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des composés selon l'invention se trouve associé à des excipients pharmaceutiquement acceptables, solides ou liquides, adaptés à la constitution de formes orale, oculaire ou nasale, ou avec des excipients adaptés à la constitution de formes d'administration rectale, ou encore avec des excipients gélatineux pour l'administration vaginale. Elle concerne aussi des compositions liquides isotoniques contenant l'un au moins des produits selon l'invention, adaptés à l'administration sur les muqueuses, notamment oculaire ou nasale. Elle concerne enfin des compositions formées de gaz liquéfiés pharmaceutiquement acceptables, du type "propellant", dans lesquels les produits selon l'invention sont dissous ou maintenus en suspension, et dont la détente provoque la dispersion en un aérosol.

L'invention consiste également en un procédé visant à renforcer les défenses immunitaires de l'hôte, consistant à lui administrer une dose efficace de l'un au moins des produits selon l'invention, sous l'une des formes d'administration qui ont été évoquées ci-dessus. A titre d'exemple de doses susceptibles d'induire une action, on mentionnera des doses de 10 à 1000 $\mu$g par kg de corps, par exemple de 50 $\mu$g, lorsque l'administration est effectuée par voie parentérale, ou encore d'une dose de 200 à 20000 $\mu$g par kg de corps, par exemple de 1000 $\mu$g, pour d'autres modes d'administration, tels que par exemple la voie orale.

D'autres caractéristiques de l'invention apparaîtront au cours de la description d'exemples de préparation de produits selon l'invention ainsi que des essais mettant en évidence les propriétés de ces produits.

Les abréviations utilisées dans la suite de la description ont les significations suivantes:

Ala : alanine

Gln : glutamine

Mur-NAc : acide N-acétyl-muramique

BOC : t-butyloxycarbonyle

OMe : ester méthylique

OBu : ester butylique

| | | |
|---|---|---|
| OSu | : | ester succinimidique |
| Bzl | : | benzyl |
| Bzi | : | benzylidène |
| Z | : | benzyloxycarbonyl |

1°) Synthèse de l'ester méthylique du N-acétyl-muramyl-L-alanyl-D-glutamine

a) t-butyloxycarbonyle de l'ester méthylique de D-glutamine BOC—D—Gln—OMe (I)

1,5 g (6,1 mmoles) de t-butyloxycarbonyl-D-glutamine (BOC—D—Gln), préparé selon la méthode décrite par SCHNABEL (Liebigs, Ann. Chem. (1967) *702,* 188—196), sont dissous dans 60 ml de méthanol absolu. A 0°C, on ajoute goutte à goutte une solution éthérée de diazométhane jusqu'à persistance de la coloration jaune. Le mélange réactionnel est laissé sous agitation à 0°C pendant 10 minutes, puis à température ordinaire pour un temps identique. La progression de la réaction est vérifiée par chromatographie sur couche mince de gel de silice dans les systèmes de solvants n-butanol-acide acétique-eau (4:1:5 phase supérieure), n-butanol-pyridine-acide acétique-eau (30:20:6:24) et chloroforme-méthanol (5:1). L'excès de diazométhane est détruit par addition d'acide acétique glacial et le mélange réactionnel est concentré à sec. Le résidu est repris dans un minimum d'acétate d'éthyle et précipité à l'éther de pétrole. Son point de fusion est 86—90°C et son pouvoir rotatoire $|\alpha|_D^{25} = +24,7°$ (méthanol).

On obtient 1,417 g de BOC—D—Gln—OMe, soit un rendement de 89,7%.

L'analyse élémentaire de ce produit est pour $C_{11}H_{20}N_2O_5$ (260, 29)

| | | C | H | N |
|---|---|---|---|---|
| calculé | : | 50,76 | 7,75 | 10,76 |
| trouvé | : | 50,62 | 7,75 | 10,82 |

b) Chlorhydrate de l'ester méthylique de D-glutamine HCl, D—Gln—OMe (II)

1,4 g (5,4 mmoles) de (I) sont traités par 15 ml d'une solution normale d'acide chlorhydrique dans l'acide acétique glacial. Après 30 minutes à température ordinaire, le mélange réactionnel est concentré à sec et l'huile obtenue est desséchée sous vide en présence de KOH.

c) Ester méthylique de la t-butyloxycarbonyl-l-alanyl-D-glutamine BOC—L—Ala—D—Gln—OMe (III)

1,86 g (6,5 mmoles) de l'ester succinimidique de t-butyloxycarbonyl-alanine (BOC—Ala—OSu), préparé selon ANDERSON et al. (J. Am. Chem. Soc. (1964) *86,* 1839—1842), sont ajoutés à une solution de 1,2 g (5,4 mmoles) de (II) et de 0.6 ml (5,4 mmoles) de N-méthylmorpholine dans 25 ml de diméthylformamide. Après une nuit à température ordinaire, le mélange réactionnel est concentré à sec et chromatographié sur une colonne (27 × 3 cm) de silice, prééquilibrée dans le système chloroforme-isopropanol-acide acétique (100:0,5:0,2), par élution avec le système chloroforme-isopropanol-acide acétique (100:5:2). Les fractions contenant le produit sont réunies et concentrées à sec. Par cristallisation dans le mélange isopropanol-éther isopropylique, on obtient 1,035 g de (III). Son point de fusion est 115—116°C et son pouvoir rotatoire $|\alpha|_D^{25} = -9°$ (méthanol).

L'analyse élémentaire de ce produit est pour $C_{14}H_{25}N_3O_6$ (331, 37)

| | | C | H | N |
|---|---|---|---|---|
| calculé | : | 50,74 | 7,60 | 12,68 |
| trouvé | : | 50,79 | 7,40 | 12,42 |

d) Chlorhydrate de l'ester méthylique de L-alanyl-D-glutamine HCl, L—Ala—D—Gln—OMe (IV)

332 mg (1 mmole) de (III) sont traités par 3 ml d'une solution normale d'acide chlorhydrique dans l'acide acétique glacial. Après 30 minutes à température ordinaire, le mélange réactionnel est concentré à sec et l'huile obtenue est desséchée sous vide en présence de KOH.

e) Couplage du dérivé peptidique (IV) au dérivé protégé du résidu muramyle (1-$\alpha$-Bzl-4,6-Bzi)-Mur-NAc-L-Ala-D-Gln-OMe (V).

471,5 mg (1 mmole) de (1-$\alpha$-Bzl-4,6-Bzi)-Mur-NAc, préparés selon OSAWA et JEANLOZ (J. Org. Chem. (1965) *30,* 488), sont dissous dans 5 ml de diméthylformamide contenant 0,11 ml (1 mmole) de N-méthyl-morpholine. A cette solution refroidie à —15°C est ajouté 0,13 ml (1 mmole) de chloroformiate d'isobutyle. Après 3 à 5 minutes, une solution, refroidie à —15°C, de 268 mg (1 mmole) de (IV) et de 0,11 ml (1 mmole) de N-méthylmorpholine dans 5 ml de diméthylformamide est ajoutée à la solution précédente. Après une nuit à —15°C, on ajoute 1 ml d'une solution 2,5 M de bicarbonate de potassium. Après 30 minutes à 0°C, le produit est précipité par addition d'eau au mélange réactionnel, filtré, lavé sur le filtre avec une solution molaire de bicarbonate de potassium et séché sous vide en présence de $P_2O_5$. On obtient 620 mg (90,5%) du produit (V). Son point de fusion est 215—220°C et son pouvoir rotatoire $|\alpha|_D^{25} = +92,6°$ (diméthylformamide).

f) Ester méthylique de la N-acétyl-muramyl-L-alanyl-D-glutamine Mur—NAc—L—Ala—D—Gln—OMe (VI)

587,6 mg (0,86 mmole) de (V), dissous dans 35 ml d'acide acétique glacial, sont hydrogénés pendant 60 heures en présence de 450 mg de palladium 5% sur chabon. Après filtration du catalyseur, l'acide acétique est éliminé sous vide. Le produit est repris dans une solution 0,1 M d'acide acétique, déposé sur une colonne (10 × 1 cm) de résine échangeuse d'ions comercialisée sous le nom "Amberlite AG 50 W—X 2", et élué avec la même solution acétique. Après lyophilisation de l'éluat contenant le produit, la même procédure est suivie pour une chromatographie du produit sur résine "Amberlite AG 1 X—2". On obtient finalement 290 mg de produit lyophilisé.

Ce produit est chromatographié sur une colonne de silice préalablement équilibrée dans le mélange n-butanol-acide acétique-eau (65:10:25). Le même mélange est utilisé pour l'élution du produit. Les fractions contenant le produit pur sont réunies, extraites à l'eau, et la phase aqueuse obtenue est lyophilisée. On obtient 234,4 mg du produit. Son point de fusion est 108—112°C et son pouvoir rotatoire $|\alpha|_D^{25} = +34,3°$ (acide acétique glacial).

L'analyse élémentaire du produit est la suivante pour $C_{20}H_{34}N_4O_{11}$—1,25 $H_2$ (529, 02)

| | | C | H | N |
|---|---|---|---|---|
| calculé | : | 45,40 | 6,95 | 10,59 |
| trouvé | : | 45,46 | 6,76 | 10,56 |

2°) Synthèse de l'ester n-butylique de N-acétyl-muramyl-L-alanyl-D-glutamine

a) Z—Ala—D—Gln (I)

2,4 g (16,4 mM) de D-glutamine sont solubilisés à chaud dans 35 ml d'eau. A température ambiante, on ajoute 2,3 ml (16,8 mM) de triéthylamine et 4,62 g (14,4 mM) de Z—L—Ala—OSu, en solution dans 70 ml de tétrahydrofuranne (THF) anhydre. La réaction se fait à 4°C pendant 48 heures. On ajoute 0,5 ml (3,85 mM) de diméthylaminopropylamine.

Après 1 heure à température ordinaire, le mélange réactionnel est dilué par 65 ml d'eau, puis acidifié à pH 2,2—3 avec de l'HCl 4 N, à 0°C. Le THF est éliminé sous vide.

Le produit précipité. Après une nuit à 4°C, il est filtré et lavé avec un minimum d'eau glacée. Il est cristallisé dans l'éthanol entre chaud et froid.

On obtient 2,87 g de produit, soit un rendement de 62%. Son point de fusion est 179—182°C, et son pouvoir rotatoire $|\alpha|_D^{20} = -13,3°$ (méthanol).

L'analyse élémentaire n est: pour $C_{16}H_{21}N_3O_6$ (351, 37)

| | | C | H | N |
|---|---|---|---|---|
| calculé | : | 54,69 | 6,02 | 11,96 |
| trouvé | : | 54,18 | 5,8 | 11,77 |

b) Z—Ala—D—Gln—O—n—Bu (II)

350 mg (1 mM) de (I), dissous dans 15 ml de THF et 5 ml d'$H_2O$, sont transformés en sel de cesium par addition d'une solution aqueuse à 20% de $Cs_2CO_3$ (1 mM). Après concentration à sec, puis séchage par évaporation avec du diméthylformamide, plusieurs fois, et par dessication sous vide (dessicateur avec $P_2O_5$), on dissout le résidu sec dans 30 ml de diméthylformamide, et on ajoute 0,12 ml (1,1 mM) de 1-bromo-n-butane.

Après 20 heures, on vérifie, par chromatographie sur couche mince de gel de silice dans le système acétate-pyridine-acide acétique-eau (6:2:0,6:1), que la réaction est terminée. Sinon, on ajoute à nouveau 0,06 ml (0,55 mM) de 1-bromo-n-butane et on laisse réagir à nouveau 20 heures. Le mélange réactionnel est alors concentré au minimum et le produit précipité à l'eau.

On obtient 332 mg de produit, soit un rendement de 81,5%. Son point de fusion est 148—150°C.

L'analyse élémentaire en est: pour $C_{20}H_{29}N_3O_6$ (407, 47)

| | | C | H | N |
|---|---|---|---|---|
| calculé | : | 58,95 | 7,17 | 10,31 |
| trouvé | : | 58,91 | 7,12 | 10,26 |

c) L—Ala—D—Gln—O—n—Bu (III)

330 mg (0,8 mM) de (II), dissous dans 30 ml d'acide acétique glacial, sont hydrogénés pendant 4 heures, en présence de 330 mg de Pd (5%) sur charbon et de 1 ml (1 mM) de HCl N. On vérifie, par chromatographie sur couche mince de gel de silice dans le système acétate d'éthyle-pyridine-acide acétique-eau (6:2:0,6:1), que l'hydrogénation est terminée, puis on filtre le catalyseur et on concentre le solvant. L'huile résiduelle est séchée soigneusement sous vide (dessicateur, avec $P_2O_5$) et utilisée telle quelle à l'étape suivante.

On obtient 254 mg de produit, soit un rendement de 100%.

d) Mur—NAc(1-bzl-4,6-O-Bzi)—L—Ala—D—Gln—OBu (IV)

La préparation de ce dérivé est effectuée classiquement via la méthode aux anhydrides mixtes.

A partir de 0,9 mM de Mur—NAc(1-bzl-4,6-O-Bzi) et 0,8 mM de (III), on obtient un rendement 81,2%. Le point de fusion du produit est 220—235°C et son pouvoir rotatoire $|\alpha|_D^{20} = +82,6°$ (diméthylformamide).

L'analyse élémentaire du produit est: pour $C_{37}H_{50}N_4O_{11}$ (726, 84)

| | C | H | N |
|---|---|---|---|
| calculé : | 61,14 | 6,93 | 7,71 |
| trouvé : | 61,77 | 7,03 | 7,03 |

e) Mur—NAc—L—Ala—D—Gln—O—n—Bu (V)

472 mg (0,6 mM) de (IV), dissous dans 30 ml d'acide acétique glacial, sont hydrogénés pendant 40 heures, en présence de 470 mg de Pd (5%) sur charbon. Aprés contrôle chromatographique sur plaque de gel de silice dans de système $CHCl_3$—MeOH (50—15), le catalyseur est filtré et le filtrat concentré.

Le résidu huileux est repris dans $CHCl_3$—MeOH (50:15) et chromatographié sur une colonne de silice 60 (33 g) avec $CHCl_3$—MeOH (50:15). Les fractions contenant le produit sont concentrés à sec; le résidu est repris dans l'eau, puis lyophilisé après ultrafiltration.

On obtient 157 mg de produit, soit un rendement de 48%. Son pouvoir rotatoire est $|\alpha|_D^{20} = +34,8°$ (acide acétique).

L'analyse élémentaire du produit est: pour $C_{23}H_{40}N_4O_{11}$ 1/3 $H_2O$

| | C | H | N |
|---|---|---|---|
| calculé : | 48,24 | 7,50 | 9,78 |
| trouvé : | 48,26 | 7,08 | 9,74 |

3°) Synthèse de l'ester n-décylique de N-acétyl-muramyl-L-alanyl-D-glutamine

a) Ce produit est préparé selon la méthode décrite ci-dessus pour l'ester n-butylique de N-acétyl-muramyl-L-alanyl-D-glutamine.

Le produit obtenu dans ces conditions présente un pouvoir rotatoire de $|\alpha|_D^{20} = +30°$ (acide acétique glacial)

Son analyse élémentaire est: pour $C_{29}H_{52}N_4O_{11}$—0,15 $CHCl_3$ (650, 64)

| | C | H | N |
|---|---|---|---|
| calculé : | 53,81 | 8,08 | 8,61 |
| trouvé : | 53,97 | 8,06 | 8,47 |

b) Le même produit a été synthétisé selon une deuxième séquence.

492,5 mg (1 mM) de Mur—NAc—L—Ala—D—Gln, dissous dans 15 ml de THF et 5 ml d'$H_2O$, sont transformés en sel de cesium par addition d'une solution aqueuse à 20% de $Cs_2CO_3$ (1 mM). Après concentration à sec, puis séchage par évaporation avec du diméthylformamide, plusieurs fois, et par dessication, on dissout le résidu dans 30 ml de diméthylformamide, et on ajoute 0,13 ml (1,1 mM) de 1-bromo-n-décane. Après 20 heures, le mélange réactionnel est concentré à sec. Le produit, après chromatographie sur colonne de silice dans le mélange chloroforme-méthane (50:15), est obtenu par précipitation dans méthanol-éther.

Son pouvoir rotatoire est $|\alpha|_D^{20} = +29°$ (acide acétique glacial).

L'analyse élémentaire du produit est: pour $C_{29}H_{52}N_4O_{11}$—0,25 $CHCl_3$ (661, 9)

| | C | H | N |
|---|---|---|---|
| calculé : | 53,08 | 7,96 | 8,47 |
| trouvé : | 53,2 | 8,0 | 8,50 |

4°) Synthèse de l'ester n-propylique de N-acétyl-muramyl-L-alanyl-D-glutamine

Le produit est préparé selon la methode décrité pour l'ester n-butylique de N-acétyl-muramyl-L-alanyl-D-glutamine.

Le pouvoir rotatoire du produit obtenu est $|\alpha|_D^{20} = +32,3$ (acide acétique glacial).

L'analyse élémentaire est: pour $C_{22}H_{38}N_4O_{11}$ 0,6 $CHCl_3$ 1$CH_3COOH$(664, 65)

| | C | H | N |
|---|---|---|---|
| calculé : | 44,4 | 6,4 | 8,4 |
| trouvé : | 44,5 | 6,3 | 8,4 |

5°) Synthèse de l'ester n-hexylique de N-acétyl-muramyl-L-alanyl-D-glutamine

Le produit est préparé selon la méthode décrite pour l'ester n-butylique de N-acétyl-muramyl-D-glutamine.

Le pouvoir rotatoire du produit obtenu est $|\alpha|_D^{20} = +29.5$ (acide acétique glacial).

L'analyse élémentaire est: pour $C_{25}H_{44}N_4O_{11}$ 0,3 $CHCl_3$ (612, 47)

| | | C | H | N |
|---|---|---|---|---|
| calculé | : | 49,6 | 7,3 | 9,1 |
| trouvé | : | 49,4 | 7,4 | 9,1 |

Propriétés pharmacologiques

1°) Toxicité

On a étudié la toxicité des produits selon l'invention par administration parentérale notamment sur des lapins. On a constaté que les doses toxiques sont d'un ordre de grandeur très sepérieur à celui des doses auxquelles ces produits manifestent leur activité. Ainsi, ces produits sont bien tolérés chez le lapin pour des doses égales ou supérieures à 5 mg/kg d'animal.

2°) Pyrogénicité

Au cours des essais sur les lapins, on a suivi les variations maximum de température pendant les trois heures suivant l'administration. Pour des doses aussi élevées que 5 mg/kg ou plus de produit, on ne constate aucune variation significative de température. Ainsi, l'élévation moyenne de température pour l'administration à des lapins, pour différents esters de Mur—NAc—L—Ala—D—Gln est:

ester méthylique à 5 mg/kg 0,53°C

ester butylique à 10 mg/kg 0,30°C

ester décylique à 10 mg/kg 0,23°C

On peut donc considérer que, aux doses actives telles qu'elles ressortent des essais reportés ci-après, ce produit est totalement apyrogène.

3°) Caractère adjuvant en phase aqueuse

Dans la série d'essais dont les résultats sont indiqués ci-après, on a étudié l'influence du principe actif selon l'invention sur le taux des anticorps anti-albumine dans les conditions suivantes.

Des groupes de 8 souris Swiss âgées de deux mois reçoivent, par injection sous-cutanée, 0,5 mg d'antigène constitué par de l'albumine de sérum bovin (BSA) avec ou sans la substance essayée dans une solution saline isotonique. Cette dose élevée d'antigène, parce qu'elle se situe à la limite de la dose paralysante vis-à-vis de la réponse immunitaire, entraîne, de ce fait, une réponse faible ou nulle à l'antigène seul chez les témoins; elle constitue donc un critère sévère pour mettre en évidence l'activité d'une substance adjuvante. Trente jours plus tard, les souris reçoivent, par la même voie d'administration, un rappel contenant 0,1 mg du même antigène.

A titre de comparaison, on a étudié simultanément l'effet adjuvant de la 2-(2-acétamido-2-déoxy-3-O-D-glucopyranosyl)-D-propionyl-L-alanyl-D-isoglutamine (MDP) connue pour ses propriétés adjuvantes.

Le taux d'anticorps est déterminé par hémagglutination passive en utilisant des hématies de mouton traitées à la formaline et recouvertes de l'antigène étudié selon la methode décrite par A.A. HIRATA et M.W. BRANDISS (J. Immunol., *100,* 641—648, 1968). On effectue les prélèvements respectivement après la première injection d'antigène et après l'injection de rappel pour déterminer les réponses primaires et secondaires.

Les résultats de ces essais sont donnés dans le tableau suivant. Les titres en anticorps expriment la dilution sérique maximale agglutinant une quantité donné d'hématies de mouton.

Tableau 1

| | | Titre d'anticorps | |
|---|---|---|---|
| | | Réponse primaire | Réponse secondaire |
| Témoins BSA | | <3 | 16 ± 16 |
| BSA + MDP | 100 µg | 50 | 300 ± 100 |
| BSA + Mur-NAc-L-Ala-D-Glu($NH_2$)-$OCH_3$ | 100 µg | 100 | 500 ± 150 |
| BSA + Mur-NAc-L-Ala-D-Glu($NH_2$)-$OC_4H_9$ | 100 µg | 12 | 600 ± 200 |
| BSA + Mur-NAc-L-Ala-D-Glu($NH_2$) | 100 µ | 25 | 200 ± 90 |

Ces résultats montrent que les esters méthylique et butylique, administrés en solution saline isotonique, provoquent un important accroissement des taux d'anticorps formés. L'effet apparaît même supérieur à celui que l'on observe chez les sujets traités avec le MDP.

4°) Caractère adjuvant du Mur—NAc—L—Ala—D—Glu($NH_2$)—$OCH_3$ en présence d'une phase huileuse

Dans ces essais, on suit l'acroissement du taux d'anticorps spécifiques d'un antigène donné lorsque ce dernier est injecté, avec ou sans le composé adjuvant selon l'invention, au sein d'une émulsion eau dans l'huile.

Les essais sont effectués sur les lots de 6 cobayes Hartley femelles de 350 g. L'administration est faite par injection intradermique dans le coussinet plantaire de chacune des pattes postérieures. L'ovalbumine (constituant l'antigêne) à raison de 1 mg est préparée dans 0,1 ml d'une émulsion de solution isotonique saline, dans une phase huileuse constituée par l'adjuvant incomplet de Freund (FIA). Le composé selon l'invention est administré à raison de 0,1 mg additionné dans l'emulsion contenant le FIA.

Comme précédemment, à titre de compariason, on effectue un essai avec du MDP à la place du produit selon l'invention.

Dix-huit jours après cette immunisation, on recherche d'éventuelles réactions d'hypersensibilité retardée à l'antigène en injectant par voie intradermique 0,01 mg d'ovalbumine sur le flanc des animaux, et l'on observe, 48 heures après, la réaction au point d'injection. On mesure de diamètre en millimètres de la réaction ainsi provoquée.

Vingt et un jours après l'injection, les animaux sont saignés. Sur le sérum recueilli, on mesure la teneur en anticorps spécifiques de l'ovalbumine par précipitation du complexe anticorps-antigène dans la zone d'équivalence. La quantité d'azote protéique contenue dans ce précipité est évaluée suivant la méthode de Folin. Les valeurs moyennes des teneurs en anticorps sont indiquées dans le tableau de résultats. Ces valeurs expriment la quantité, en microgrammes, d'azote précipitable par l'antigène, par millilitre de sérum.

Les résultats de ces essais sont reportés dans la tableau 2 suivant.

Tableau 2

| Composition de l'émulsion contenant l'antigène | | Anticorps sériques ($\mu g/ml$) | Test cutané (diamètre en mm) |
|---|---|---|---|
| FIA | | 800 | 0 |
| FIA + MDP | (100 $\mu g$) | 5.000 | 15 |
| FIA + Mur-NAc-L-Ala-D-Glu($NH_2$)-$OCH_3$ | (100 $\mu g$) | 6.000 | 17 |
| FIA + Mur-NAc-L-Ala-D-Glu($NH_2$)-$OC_4H_9$ | | 2.000 | 8 |

Ces résultats montrent que les esters méthylique et butylique, administrés dans une émulsion huileuse, permettent d'accroître de façon très importante le taux d'anticorps formés en réponse à l'injection d'antigène, et qu'il induit une réaction d'hypersensibilité retardée vis-à-vis du même antigène, ces deux réactions étant au moins égales à celles que l'on observe avec le MDP.

5°) Activité anti-infectieuse vis-à-vis de Klebsiella

Le protocole des essais est décrit dans l'article CHEDID L. et col., Proc. Natl. Acad. Sci. USA, 1977, *74*: 2089.

On a ainsi établi de façon préalable un mode expérimental permettant de mettre en évidence le caractère anti-infectieux des produits. On a montré qu'une dose de 1 à $2.10^4$ *Klebsiella pneumoniae,* injectée par voie intramusculaire à des souris, entraîne le décès progressif d'une part importante, sinon de la totalité, des animaux dans la semaine suivante l'inoculation. Après huit jours, la survie des animaux est définitivement acquise.

On a suivi la survie de groupes de souris inoculées dans les conditions indiquées ci-dessus et traitées à l'aide de produits selon l'invention.

Pour ces essais, on a utilisé des souris hybrides (C57B1/6 × AKR)F1 élevées à l'INSTITUT PASTEUR, à partir de souches provenant de l'élevage du C.N.R.S. à ORLEANS.

L'infection par *Klebsiella pneumoniae,* souche de type capsulaire 2, biotype d, est faite à partir d'une culture de 16 heures en milieu pour pneumocoques (n° 53515, INSTITUT PASTEUR). La dose infectante est $2.10^4$ *Klebsiella*; elle est administrée par voie intramusculaire.

L'administration du produit testé est réalisée par voie intraveineuse dans 0,2 ml du soluté physiologique apyrogène, les témoins recevant le soluté seul. Elle est réalisée 24 heures avant l'inoculation.

Dans les conditions de ces essais, les esters butylique et décylique du Mur—NAc—L—Ala—D—Glu montrent une activité anti-infectieuse, ce qui se traduit par un pourcentage de survivants plus élevé que celui du groupe témoin.

Tableau 3

| Produit | Nombre souris | Survie % | | | % Protection |
|---|---|---|---|---|---|
| | J0 | J3 | J5 | J10 | |
| Témoins | 24 | 29 | 12,5 | 12,5 | |
| Mur-NAc-L-Ala-D-Glu($NH_2$)-$OC_4H_9$ | 24 | 92 | 75 | 75 | 63 |

6°) Absence d'influence vis-à-vis des mécanismes de coagulation

On sait, par des études récentes, que des composés présentant des propriétés adjuvantes comme

les LPS peuvent avoir des effets secondaires. On a ainsi constaté chez le lapin que l'administration de LPS a tendance à raccourcir le temps de coagulation. Les raisons de ce phénomène ne sont pas parfaitement connues; néanmoins, il a semblé utile de déterminer l'effet des produits selon l'invention sur la coagulation dans les mêmes conditions.

Pour ces essais, on utilise le test décrit par LERNER et col. dans Thrombosis Research, vol. 11, pp. 253—261, 1977, "Endotoxin induced disseminated intravascular clotting: evidence that it is mediated by neutrophile production of tissue factor".

On divise le sang total qui a été prélevé en deux parties dont l'une est centrifugée pour obtention de plasma déplaquetté (PDP).

1 ml de sang total est mis à incuber dans un tube plastique en présence de 100 $\mu$g de produit à tester.

On prépare par ailleurs des tubes contenant 0,1 ml de PDP auxquels on ajoute toutes les heures, de 0 à 5 heures, 0,1 ml du mélange d'incubation, puis on recalcifie par 0,1 ml de chlorure de calcium 0,025 M.

On note le temps de coagulation et on en déduit l'activité procoagulante en fonction du raccourcissement du temps de coagulation par rapport au témoin, eau physiologique. On compare le résultat avec celui d'un étalon de référence hautement procoagulant, le LPS.

Les résultats de ces essais figurent dans le tableau suivant. Les temps de coagulation sont exprimés en secondes.

On constate que les esters butylique, méthylique... de Mur—NAc—L—Ala—D—Glu, contrairement au LPS, n-accélèrent pas la coagulation.

Tableau 4

| Incubation h | Eau | LPS | Ester méthyliq. | Ester butylique |
|---|---|---|---|---|
| 0 | 131 | 129 | 125 | 129 |
| 2 | 129 | 118 | 118 | 120 |
| 3 | 122 | 98 | 109 | 122 |
| 4 | 119 | 88 | 110 | 114 |
| 5 | 113 | 77 | 104 | 108 |

**Revendications pour l'Etat contractants: BE CH DE FR GB IT LU NL SE**

1. Composé répondant à la formule générale:

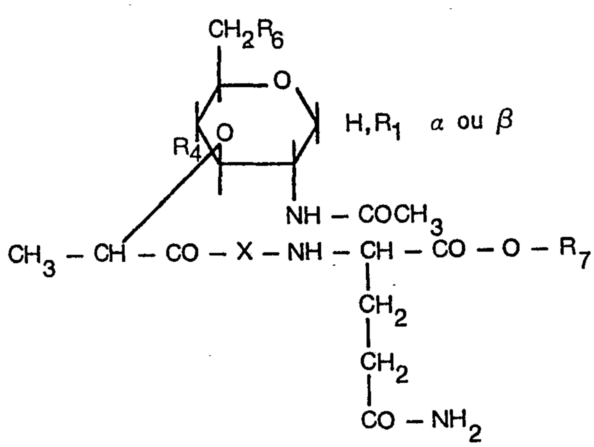

dans laquelle les substituants $R_1$, $R_4$, $R_6$, X et $R_7$ ont respectivement les significations suivantes:

$R_1$ est —$NH_2$, —OH, —NY ou —OY, Y représentant un groupe substitué ou non par un groupe amino, ce groupe étant choisi parmi alcoyle, aryle ou alcoyle-aryle portant un maximum de 10 atomes de carbone;

$R_4$ est hydroxyle, acyloxy ou alcoxy ayant au plus 4 atomes de carbone, ou monosuccinyle;

$R_6$ est $—NH_2$, —OH, —NHZ ou —OZ, Z étant un radical linéaire ou ramifié formé par acyle ou alkyle contenant de 1 à 90 atomes de carbone et portant, le cas échéant, des substituants hydroxyle, carboxyle, carbonyle, amino, cyclopropyle ou méthoxyle;

X est un résidu aminoacyle du groupe comprenant: L-alanyle, L-arginyle, L-asparagyle, L-aspartyle, L-cystéinyle, L-glutaminyle, L-glutamyle, glycyle, L-histidyle, L-hydroxy-propyle, L-isoleucyle, L-leucyle, L-lysyle, L-méthionyle, L-phénylalanyle, L-propyle, L-séryle, L-thréonyle, L-tryptophanyle, L-tyrosyle et L-valyle,

$R_7$ est un groupe alcoyle linéaire ou ramifié, aryle ou alcoyl-aryle comprenant au plus 10 atomes de carbone.

2. Composé selon la revendication 1, caractérisé en ce que X est un résidu L-alanyle, L-séryle ou glycyle.

3. Composé selon la revendication 1, caractérisé en ce que X est un résidu aminoacyle du groupe comprenant: L-prolyle, L-thréonyle et L-valyle.

4. Composé selon l'une quelconque des revendications 1 à 3, caractérisé en ce que $R_7$ est un radical $—CH_3$, $—C_2H_5$ ou $—C_3H_7$.

5. Composé selon l'une quelconque des revendications 1 à 3, caractérisé en ce que $R_7$ est un radical alcoyle contenant 4 carbones.

6. Composé selon l'une quelconque des revendications 1 à 5, caractérisé en ce que $R_1$ est —OH.

7. Composé selon l'une quelconque des revendications 1 à 6, caractérisé en ce que $R_4$ est —OH, $—O—COCH_3$ ou $—O—CO—(CH_2)_2—CO_2H$.

8. Composé selon la revendication 1, caractérisé en ce que $R_6$ est une fonction amine ou un ester, dont le résidu acyle contient de 1 à 6 atomes de carbone.

9. Composé selon la revendication 1, caractérisé en ce que $R_6$ est un groupe monosuccinyle.

10. Composé selon la revendication 1, caractérisé en ce que $R_1$, $R_4$, $R_6$ sont simultanément —OH, X est L-alanyle et $R_7$ est $—CH_3$, $—C_2H_5$ ou $—C_3H_7$.

11. Composé selon la revendication 1, caractérisé en ce que le groupe $R_7$ comporte au moins 4 atomes de carbone.

12. Composé selon la revendication 1, caractérisé en ce que le résidu X est un L-alanyle.

13. Composé selon la revendication 1, caractérisé en ce que $R_1$, $R_4$, $R_6$ sont simultanément —OH, X est L-alanyle et $R_7$ est $C_4H_9$.

14. Composé selon la revendication 1, caractérisé en ce qu'il est constitué par l'ester n-butylique de la N-acétyl-muramyl-L-alanyl-D-glutamine.

15. Composé selon la revendication 1, caractérisé en ce que $R_1$, $R_4$, $R_6$ sont simultanément —OH, X est L-alanyle et $R_7$ est $—C_6H_{13}$ ou $—C_{10}H_{21}$.

16. Composition pharmaceutique caractérisée en ce qu'elle contient une dose efficace d'au moins un composé selon l'une quelconque des revendications 1 à 15.

17. Composition pharmaceutique selon la revendication 16, caractérisée en ce que le ou les composés selon l'invention sont associés à des véhicules ou excipients pharmaceutiquement acceptables.

18. Composition pharmaceutique selon la revendication 16 ou la revendication 17, constituée par une solution, suspension ou une forme liposome du composé selon l'invention dans un milieu injectable.

19. Composition pharmaceutique selon la revendication 16 ou la revendication 17, caractérisée en ce que le composé selon l'invention est associé à des excipients permettant l'administration par application sur les muqueuses oculaires ou des voies respiratoires, ou à des excipients pour l'administration vaginale, ou à des excipients pour l'administration rectale.

20. Composition pharmaceutique selon la revendication 16, caractérisée en ce qu'elle contient également un agent immunogène.

21. Réactif biologique comprenant au moins un composé selon l'une quelconque des revendications 1 à 15.

**Revendications pour l'Etat contractant: AT**

1. Procédé de préparation d'un composé de formule:

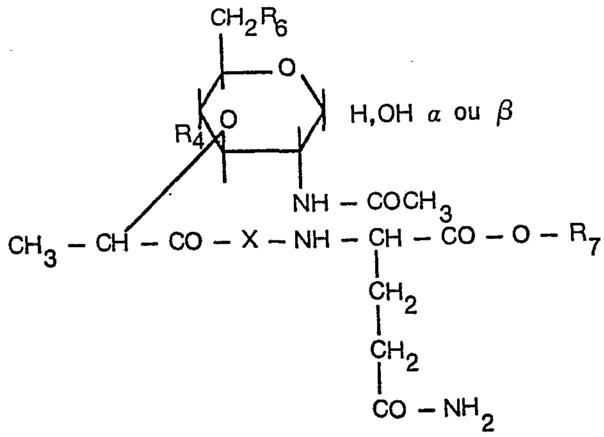

dans laquelle les substituants $R_4$, $R_6$, X et $R_7$ ont respectivement les significations suivantes:

$R_4$ est hydroxyle, acyloxy ou alcoxy ayant au plus 4 atomes de carbone, ou monosuccinyle;

$R_6$ est —$NH_2$, —OH, —NHZ ou —OZ, Z étant un radical linéaire ou ramifié formé par acyle ou alkyle contenant de 1 à 90 atomes de carbone et portant, le cas échéant, des substituants hydroxyle, carboxyle, carbonyle, amino, cyclopropyle ou méthoxyle;

X est un résidu aminoacyle du groupe comprenant: L-alanyle, L-arginyle, L-asparagyle, L-aspartyle, L-cystéinyle, L-glutaminyle, L-glutamyle, glycyle, L-histidyle, L-hydroxy-propyle, L-isoleucyle, L-leucyle, L-lysyle, L-méthionyle, L-phénylalanyle, L-propyle, L-séryle, L-thréonyle, L-tryptophanyle, L-tyrosyle et L-valyle,

$R_7$ est un groupe alcoyle linéaire ou ramifié, aryle ou alcoyle-aryle comprenant au plus 10 atomes de carbone, caractérisé en ce que l'on effectue une réaction de couplage entre, d'une part, l'acide muramique de formule:

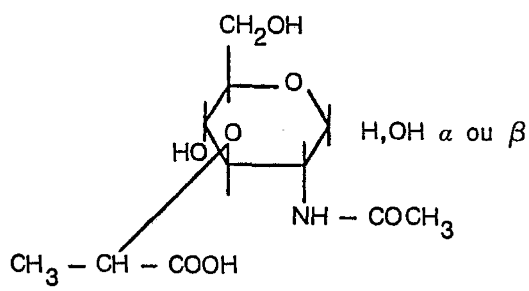

dont les hydroxyles dans les positions 1, 4 et 6 du groupe saccharidique sont bloqués temporairement par des groupes de protection, de préférence par un groupe benzylidène dans les positions 4 et 6 et par un groupe benzyle à la position 1 et, d'autre part, un dérivé peptidique de formule:

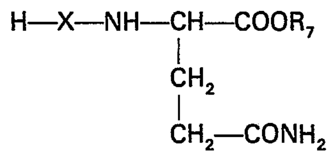

dans lequel X et $R_7$ ont les significations sus-indiquées, ou avec le dérivé peptidique correspondant dont la fonction $\alpha$-carboxylique de son groupe glutamine est libre, le groupe ester étant introduit a posteriori, les groupes de protection étant ensuite éliminés soit par hydrogénation catalytique, soit par action de l'acide acétique, puis hydrogénation catalytique, et, pour obtenir ceux des produits dans lesquels les groupes OH en la position 6 seulement ou à la fois en les positions 4 et 6 sont acylés, à faire réagir les produits précédemment obtenus avec un faible excès de l'anhydride ou le chlorure de l'acide carboxylique correspondant dans le premier cas, pour produire l'acylation sélective en position 6 du résidu saccharidique ou, dans le second cas, à le faire réagir avec un excès de l'anhydride carboxylique, pour produire la diacylation des deux hydroxyles dans les positions 4 et 6 du résidu saccharidique, lesdites réactions d'acylation pouvant être effectuées avant ou après le couplage avec le dérivé peptidique.

2. Procédé selon la revendication 1, caractérisé en ce que dans le dérivé peptidique mis en oeuvre X est L-alanyle et $R_7$ est méthyle.

3. Procédé selon la revendication 1, caractérisé en ce que dans le dérivé peptidique mis en oeuvre X est L-alanyle et $R_7$ est n-butyle.

**Patentansprüche für die Vertragsstaaten: BE CH DE FR GB IT LU NL SE**

1. Verbindung der allgemeinen Formel

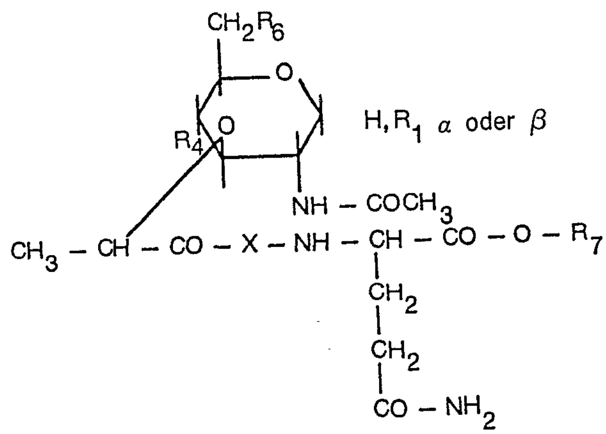

worin die Substituenten $R_1$, $R_4$, $R_6$, X und $R_7$ die folgenden Bedeutungen besitzen:

$R_1$ bedeutet —$NH_2$, —OH, —NY oder —OY, worin Y für eine gegebenenfalls durch eine Aminosäure substituierte Gruppe steht, die ausgewählt ist aus Alkyl, Aryl oder Alkyl-Aryl mit maximal 10 Kohlenstoffatomen;

$R_4$ bedeutet Hydroxy, Acyloxy oder Alkoxy mit höchstens 4 Kohlenstoffatomen oder Monosuccinyl;

$R_6$ bedeutet —$NH_2$, —OH, —NHZ oder —OZ, worin Z für einen geradkettigen oder verzweigten Rest steht, der durch Acyl oder Alkyl mit 1 bis 90 Kohlenstoffatomen gebildet ist und gegebenenfalls Hydroxy-, Carboxy, Carbonyl-, Amino-, Cyclopropyl- oder Methoxy-Substituenten trägt;

X bedeutet einen Aminoacylrest aus der Gruppe: L-Alanyl, L-Arginyl, L-Asparagyl, L-Aspartyl, L-Cysteinyl, L-Glutaminyl, L-Glutamyl, Glycyl, L-Histidyl, L-Hydroxy-propyl, L-Isoleucyl, L-Leucyl, L-Lysyl, L-Methionyl, L-Phenylalanyl, L-Propyl, L-Seryl, L-Threonyl, L-Tryptophanyl, L-Tyrosyl und L-Valyl;

$R_7$ bedeutet eine geradkettige oder verzweigte Alkylgruppe, eine Arylgruppe oder eine Alkyl-Arylgruppe mit höchstens 10 Kohlenstoffatomen.

2. Verbindung nach Anspruch 1, dadurch gekennzeichnet, daß X einen L-Alanyl, L-Seryl- oder Glycyl-Rest darstellt.

3. Verbindung nach Anspruch 1, dadurch gekennzeichnet, daß X einen Aminoacylrest aus der L-Prolyl, L-Threonyl und L-Valyl umfassenden Gruppe darstellt.

4. Verbindung nach einem der Ansprüche 1 bis 3, dadurch gekennzeichnet, daß $R_7$ eine Gruppe der Formeln —$CH_3$, —$C_2H_5$ oder —$C_3H_7$ darstellt.

5. Verbindung nach einem der Ansprüche 1 bis 3, dadurch gekennzeichnet, daß $R_7$ einen Alkylrest mit 4 Kohlenstoffatomen darstellt.

6. Verbindung nach einem der Ansprüche 1 bis 5, dadurch gekennzeichnet, daß $R_1$ —OH bedeutet.

7. Verbindung nach einem der Ansprüche 1 bis 6, dadurch gekennzeichnet, daß $R_4$ —OH, —O—$COCH_3$ oder —O—CO—$(CH_2)_2$—$CO_2H$ darstellt.

8. Verbindung nach Anspruch 1, dadurch gekennzeichnet, daß $R_6$ eine Aminogruppe oder eine Estergruppe, deren Acylrest 1 bis 6 Kohlenstoffatome enthält, darstellt.

9. Verbindung nach Anspruch 1, dadurch gekennzeichnet, daß $R_6$ eine Monosuccinylgruppe bedeutet.

10. Verbindung nach Anspruch 1, dadurch gekennzeichnet, daß $R_1$, $R_4$, $R_6$ gleichzeitig —OH, X L-Alanyl und $R_7$ —$CH_3$, —$C_2H_5$ oder —$C_3H_7$ bedeuten.

11. Verbindung nach Anspruch 1, dadurch gekennzeichnet, daß die Gruppe $R_7$ mindestens 4 Kohlenstoffatome aufweist.

12. Verbindung nach Anspruch 1, dadurch gekennzeichnet, daß der Rest X ein L-Alanylrest ist.

13. Verbindung nach Anspruch 1, dadurch gekennzeichnet, daß $R_1$, $R_4$, $R_6$ gleichzeitig —OH, X L-Alanyl und $R_7$ —$C_4H_9$ bedeuten.

14. Verbindung nach Anspruch 1, dadurch gekennzeichnet, daß sie der n-Butylester von N-Acetyl-muramyl-L-alanyl-D-glutamin ist.

15. Verbindung nach Anspruch 1, dadurch gekennzeichnet, daß $R_1$, $R_4$, $R_6$ gleichzeitig —OH, X L-Alanyl und $R_7$ $—C_6H_{13}$ oder $—C_{10}H_{21}$ bedeuten.

16. Pharmazeutisch Zubereitung, dadurch gekennzeichnet, daß sie eine wirksame Dosis mindestens einer Verbindung nach einem der Ansprüche 1 bis 15 enthält.

17. Pharmazeutische Zubereitung nach Anspruch 16, dadurch gekennzeichnet, daß die erfindungsgemäßen Verbindungen mit pharmazeutisch annehmbaren Trägern oder Hilfsmitteln kombiniert sind.

18. Pharmazeutische Zubereitung nach Anspruch 16 oder Anspruch 17 in Form einer Lösung, einer Suspension oder einer Liposomform der erfindungsgemäßen Verbindung in einem injizierbaren Medium.

19. Pharmazeutische Zubereitung nach Anspruch 16 oder Anspruch 17, dadurch gekennzeichnet, daß die erfindungsgemäße Verbindung mit Hilfsstoffen kombiniert ist, die die Verabreichung über die Augenschleimhaut oder die Atmungswege ermöglichen, oder mit Hilfstoffen für die vaginale Verabreichung oder mit Hilfsstoffen für die rektale Verabreichung.

20. Pharmazeutische Zubereitung nach Anspruch 16, dadurch gekennzeichnet, daß sie zusätzlich ein immunogenes Mittel enthält.

21. Biologisches Reagens enthaltend mindestens eine Verbindung nach einem der Ansprüche 1 bis 15.

**Patentansprüche für den Vertragsstaat: AT**

1. Verfahren zur Herstellung einer Verbindung der Formel:

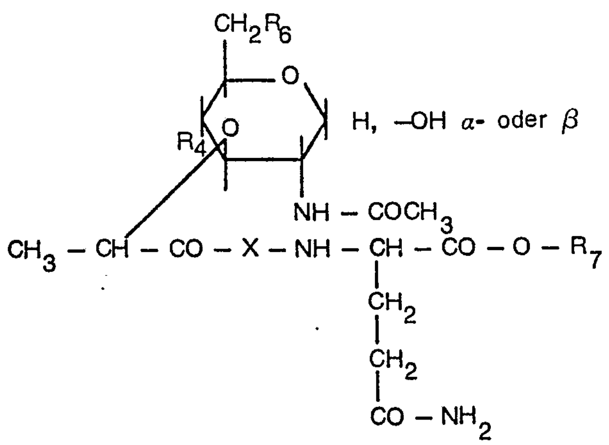

worin den Substituenten $R_4$, $R_6$, X bzw. $R_7$ die nachfolgenden Bedeutungen zukommen:

$R_4$ ist Hydroxyl Acyloxy oder Alkoxy mit höchstens 4 Kohlenstoffatomen, oder Monosuccinyl;

$R_6$ ist $—NH_2$, —OH, —NHZ oder —OZ, worin Z ein gerader oder verzweigter Acyl- oder Alkylrest mit 1 bis 90 Kohlenstoffatomen bedeutet, der gegebenenfalls Hydroxyl-, Carboxyl-, Carbonyl-, Amino-, Cyclopropyl- oder Methoxy-Substituenten trägt;

X ist ein Aminoacylrest aus der Gruppe: L-Alanyl, L-Arginyl, L-Asparagyl, L-Aspartyl, L-Cysteinyl, L-Glutaminyl, L-Glutamyl, Glycyl, L-Histidyl, L-Hydroxy-prolyl, L-Isoleucyl, L-Leucyl, L-Lysyl, L-Methionyl, L-Phenylalanyl, L-Prolyl, L-Seryl, L-Threonyl, L-Tryptophanyl, L-Tyrosyl und L-Valyl;

$R_7$ ist eine gerade oder verzweigte Alkylgruppe, Arylgruppe oder Alkarylgruppe mit höchstens 10 Kohlenstoffatomen, dadurch gekennzeichnet, daß man eine Kupplungsreaktion ausführt zwischen einerseits der Muraminsäure der Formel:

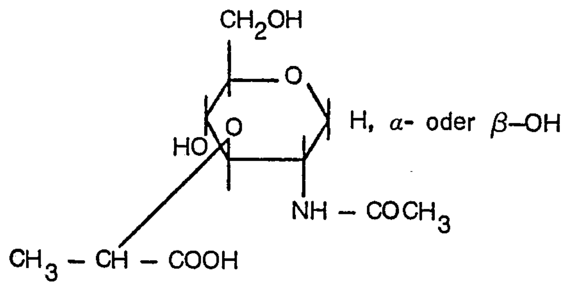

worin die Hydroxylgruppen in den 1-, 4- und 6-Stellungen der Saccharidgruppe vorübergehend durch Schutzgruppen blockiert sind, vorzugsweise durch eine Benzylidengruppe in den 4- und 6-Stellungen und durch eine Benzylgruppe in der 1-Stellung, und, anderseits, einer Peptidverbindung der Formel:

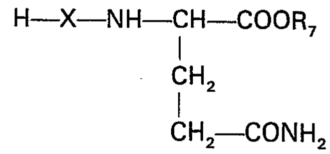

worin X und $R_7$ die vorstehend angegebenen Bedeutungen besitzen, oder mit einer korrespondierenden Peptidverbindung, worin die $\alpha$-Carboxylfunktion deren Glutamingruppe frei ist und die Estergruppe anschließend eingeführt wird, wobei die Schutzgruppen anschließend durch katalytische Hydrierung oder durch Einwirkung von Essigsäure und anschließende katalytische Hydrierung entfernt werden, und wobei zur Erzielung solcher Produkte, in welchen die OH-Gruppen in der 6-Stellung allein oder gleichzeitig in den 4- und 6-Stellungen acyliert sind, die zuvor erhaltenen Produkte im ersten Fall mit einem geringen Überschuß an dem Anhydrid oder dem Chlorid der entsprechenden Carbonsäure umgesetzt werden, um eine selektive Acylierung an der 6-Stellung des Saccharidrestes zu bewirken, oder, im zweiten Fall, mit einem Überschuß an Carbonsäureanhydrid umgesetzt werden, um die Diacylierung der beiden Hydroxylgruppen in den 4- und 6-Stellungen des Saccharidrestes zu bewirken, wobei die genannten Acylierungsreaktionen vor oder nach der Kupplung mit der Peptidverbindung ausgeführt werden können.

2. Verfahren nach Anspruch 1, dadurch gekennzeichnet, daß in der eingesetzten Peptidverbindung X für L-Alanyl steht und $R_7$ Methyl bedeutet.

3. Verfharen nach Anspruch 1, dadurch gekennzeichnet, daß in der eingesetzten Peptidverbindung X für L-Alanyl steht und $R_7$ n-Butyl bedeutet.

**Claims for the Contracting States: BE CH DE FR GB IT LU NL SE**

1. Compound of the following formula:

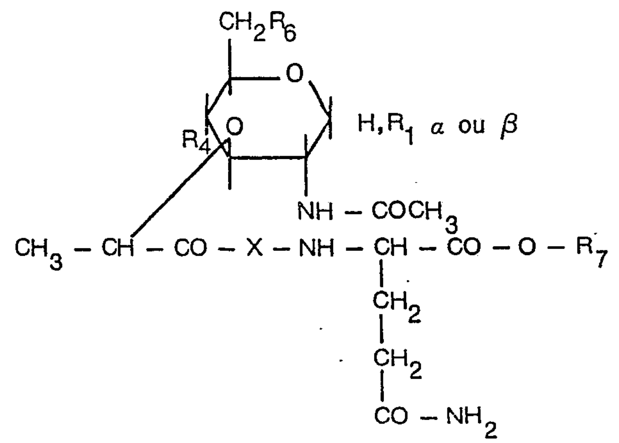

in which the substituents $R_1$, $R_4$, $R_6$, X and $R_7$ respectively have the following meanings:

$R_1$ is —$NH_2$, —OH, —NY or —OY, Y representing a group which is substituted or not by an amino group, this group being chosen from among alkyl, aryl or alkylaryl comprising a maximum of 10 carbon atoms;

$R_4$ is hydroxyl, acyloxy or alcoxy comprising at the most 4 carbon atoms, or monosuccinyl;

$R_6$ is —$NH_2$, —OH, —NHZ or —OZ, Z being a linear or branched radical constituted by acyl or alkyl containing from 1 to 90 carbon atoms and bearing, optionally, hydroxyl, carboxyl, carbonyl, amino, cyclopropyl or methoxyl substituents;

X is an aminoacyl residue of the group comprising: L-alanyl, L-arginyl, L-asparagyl, L-aspartyl, L-cysteinyl, L-glutaminyl, L-glutamyl, glycyl, L-histidyl, L-hydroxypropyl, L-isoleucyl, L-leucyl, L-lysyl, L-methionyl, L-phenylalanyl, L-propyl, L-seryl, L-threonyl, L-tryptophanyl, L-tyrosyl and L-valyl;

$R_7$ is a linear or branched alkyl, aryl or alkylaryl comprising at the most 10 carbon atoms.

2. Compound according to claim 1, characterized by the fact that X is a L-alanyl, L-seryl or glycyl residue.

3. Compound according to claim 1, characterized by the fact that X is an aminoacyl residue of the group comprising: L-prolyl, L-threonyl and L-valyl.

4. Compound according to anyone of claims 1 to 3, characterized by the fact that $R_7$ is —$CH_3$, —$C_2H_5$ or —$C_3H_7$ radical.

5. Compound according to anyone of claims 1 to 3, characterized by the fact that $R_7$ is an alkyl radical containing 4 carbon atoms.

6. Compound according to anyone of claims 1 to 5, characterized by the fact that $R_1$ is —OH.

7. Compound according to anyone of claims 1 to 6, characterized by the fact that $R_4$ is —OH, —O—$COCH_3$ or —O—CO—$(CH_2)_2$—$CO_2H$.

8. Compound according to claim 1, characterized by the fact that $R_6$ is an amino or ester function, the acyl residue of which contains from 1 to 6 carbon atoms.

9. Compound according to claim 1, characterized by the fact that $R_6$ is a monosuccinyl group.

10. Compound according to claim 1, characterized by the fact that $R_1$, $R_4$, $R_6$ are simultaneously —OH, X is L-alanyl and $R_7$ is —$CH_3$, —$C_2H_5$ or —$C_3H_7$.

11. Compound according to claim 1, characterized by the fact that $R_7$ group comprises at least 4 carbon atoms.

12. Compound according to claim 1, characterized by the fact that X residue is L-alanyl.

13. Compound according to claim 1, characterized by the fact that $R_1$, $R_4$, $R_6$ are simultaneously —OH, X is L-alanyl and $R_7$ is $C_4H_9$.

14. Compound according to claim 1, characterized by the fact that it is constituted by n-butylic ester of N-acetyl-muramyl-L-alanyl-D-glutamine.

15. Compound according to claim 1, characterized by the fact that $R_1$, $R_4$, $R_6$ are simultaneously —OH, X is L-alanyl and $R_7$ is —$C_6H_{13}$ or —$C_{10}H_{21}$.

16. Pharmaceutical composition, characterized by the fact that it contains an efficient amount of at least a compound according to any of claims 1 to 15.

17. Pharmaceutical composition according to claim 16, characterized by the fact that the compound(s) according to the invention are in association with pharmaceutically acceptable vehicles or excipients.

18. Pharmaceutical composition according to claim 16 or 17, constituted by a solution, suspension or a liposome form of the compound according to the invention in an injectable medium.

19. Pharmaceutical composition according to claim 16 or 17, characterized by the fact that the compound according to the invention is in association with excipients enabling the administration by application to the ocular mucous membranes or respiratory tracts or with excipients for vaginal administration or with excipients for rectal administration.

20. Pharmaceutic composition according to claim 16, characterized by the fact that it also contains an immunogenic agent.

21. Biological reagent comprising at least a compound according to any of claims 1 to 15.

**Claims for the Contracting State: AT**

1. Process for preparing a compound of the following formula:

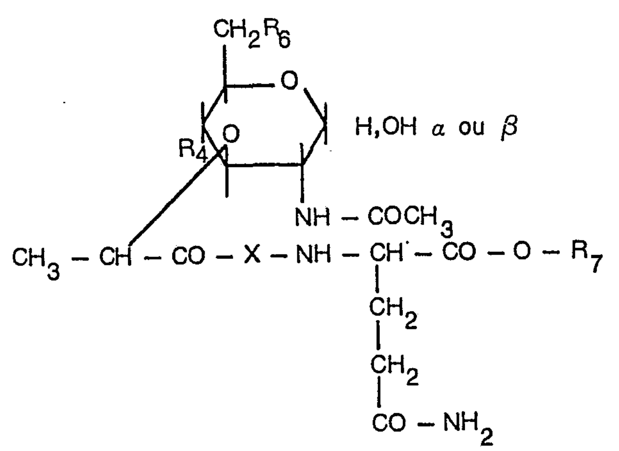

in which the substituents $R_4$, $R_6$, X and $R_7$ respectively have the following meanings:

$R_4$ is hydroxyl, acyloxy or alcoxy comprising at the most 4 carbon atoms, or monosuccinyl;

$R_6$ is —$NH_2$, —OH, —NHZ or —OZ, Z being a linear or branched radical constituted by acyl or alkyl containing from 1 to 90 carbon atoms and bearing, optionally, hydroxyl, carboxyl, carbonyl, amino, cyclopropyl or methoxy substituents;

X is an aminoacyl residue of the group comprising: L-alanyl, L-arginyl, L-asparagyl, L-aspartyl, L-cysteinyl, L-glutaminyl, L-glutamyl, glycyl, L-histidyl, L-hydroxypropyl, L-isoleucyl, L-leucyl, L-lysyl, L-methionyl, L-phenylalanyl, L-propyl, L-seryl, L-threonyl, L-tryptophanyl, L-tyrosyl and L-valyl;

$R_7$ is a linear or branched alkyl, aryl or alkylaryl comprising at the most 10 carbon atoms; characterized by the fact that a coupling reaction is carried out between:

on the other hand, a peptidic derivative of formula:

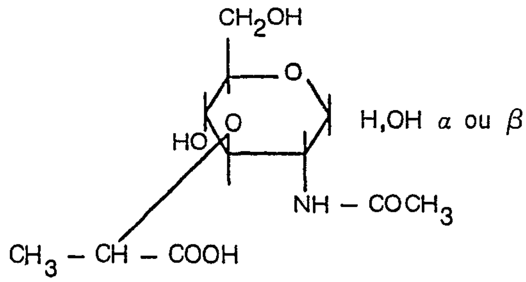

the hydroxyl groups of which in the 1, 4 and 6 positions of the saccharidic group are temporarily blocked by protective groups, preferably by a benzylidene group in the 4 and 6 positions, and by a benzyl group in the 1 position and

on the other hand, a peptidic derivative of formula:

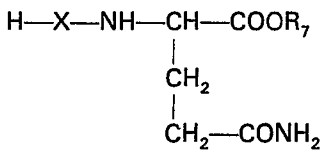

in which X and $R_7$ have the above mentioned meanings, or with the corresponding peptidic derivative in which the alpha carboxylic function of its glutamin group is free, the ester group being introduced a posteriori, the protective groups being then eliminated either by catalytic hydrogenation or by acetic acid action, then catalytic hydrogenation, and, to obtain those of the products in which the OH groups in the 6 position only or both in position 4 and 6 are acylated, to react the products which have been previously obtained with a weak excess of the anhydrid or the chloride of the corresponding carboxylic acid in the first case, to produce the selective acylation in the 6 position of the saccharidic residue, or, in the second case, to react it with an excess of carboxylic anhydrid, to produce diacylation of both hydroxyl groups in the 4 and 6 positions of the saccharidic residue, said acylation reactions being able to be carried out before or after coupling with the peptidic derivative.

2. Process according to claim 1, characterized by the fact that the peptidic derivative X which is used is L-alanyl and $R_7$ is methyl.

3. Process according to claim 1, characterized by the fact that the peptidic derivative X which is used is L-alanyl and $R_7$ is n-butyl.